# EUROPÄISCHE PATENTSCHRIFT

(11) **EP 2 596 339 B1**
(45) Veröffentlichungstag und Bekanntmachung des Hinweises auf die Patenterteilung: **23.08.2017**
(21) Anmeldenummer: 11730971.6
(22) Anmeldetag: 11.07.2011
(51) Int. Cl.: A61B 10/00, A61B 5/151, G01N 21/17, G01N 21/78, G01N 21/84, H01L 27/146

(54) **VORRICHTUNG ZUM NACHWEIS EINES ANALYTEN IN EINER KÖRPERFLÜSSIGKEIT**
DEVICE FOR DETECTING AN ANALYTE IN A BODILY FLUID
DISPOSITIF DE DÉTECTION D'UN ANALYTE DANS UN LIQUIDE CORPOREL

(30) Priorität: 20.07.2010 EP 10170088
(43) Veröffentlichungstag der Anmeldung: 29.05.2013
(73) Patentinhaber: F. Hoffmann-La Roche AG, 4070 Basel (CH); Roche Diabetes Care GmbH, 68305 Mannheim (DE)
(72) Erfinder: PETRICH, Wolfgang, 76669 Bad Schönborn (DE); WEIDNER, Frank, 68169 Mannheim (DE)
(74) Vertreter: Herzog, Fiesser & Partner Patentanwälte PartG mbB
(86) Internationale Anmeldenummer: PCT/EP2011/061783
(87) Internationale Veröffentlichungsnummer: WO 2012/010454

(56) Entgegenhaltungen:
- EP-A1- 1 582 598
- EP-A1- 1 843 148
- EP-A1- 2 151 686
- WO-A1-2010/055308
- US-B1- 6 249 593
- US-B2- 6 847 451

## Beschreibung

### Gebiet der Erfindung

Die Erfindung betrifft eine Vorrichtung zum Nachweis mindestens eines Analyten in einer Körperflüssigkeit mittels mindestens eines Testelements und vorzugsweise mittels mindestens einer Lanzette mit einer Kapillare. Weiterhin betrifft die Erfindung ein Verfahren zur Erkennung eines Auswertebereichs einer Vorrichtung zum Nachweis mindestens eines Analyten in einer Körperflüssigkeit. Derartige Vorrichtungen und Verfahren werden insbesondere in der Diagnostik eingesetzt, um einen oder mehrere Analyte, beispielsweise einen oder mehrere Metabolite wie beispielsweise Blutglucose, in Körperflüssigkeiten wie z.B. Blut oder interstitieller Flüssigkeit, qualitativ oder quantitativ nachzuweisen.

### Stand der Technik

Aus dem Stand der Technik ist eine Vielzahl von Vorrichtungen zum Nachweis mindestens eines Analyten in einer Körperflüssigkeit bekannt. Dabei werden in der Regel Testelemente eingesetzt, welche mindestens eine Testchemie aufweisen. Diese Testchemie enthält mindestens ein Nachweisreagens, welches, bei Kontakt mit dem mindestens einen Analyten, eine Analyt-spezifische Reaktion durchführt, welche beispielsweise elektrochemisch und/oder optisch nachweisbar ist.

Neben Einzelsystemen, bei welchen die Gewinnung der Probe der Körperflüssigkeit und deren Analyse getrennt erfolgen, setzen sich in jüngerer Zeit insbesondere integrierte Systeme durch. Beispielsweise setzen sich integrierte Systeme zur Blutglucosebestimmung zusammen aus der Blutgewinnung und der Glucosebestimmung. Bei der Blutgewinnung wird in einigen Systemen eine Flachlanzette mit halboffener Mikrokapillare verwendet, bei welcher die Kapillare typischerweise eine Breite von 120 µm und eine Länge von 4 mm aufweist. Nach einer Blutgewinnung durch einen Stechvorgang, beispielsweise in eine Fingerbeere, ein Ohrläppchen oder einen Unterarm, wird das in der Kapillare aufgenommene Blut häufig auf ein Testfeld des Testelements transferiert, indem die Lanzette an dieses Testfeld angenähert wird, beispielsweise auf dieses aufgedrückt wird. Hierdurch entsteht ein ebenfalls ca. 120 µm breiter Abdruck der Kapillare auf dem Testelement, beispielsweise einem streifenförmigen Testelement, welches sich, je nach Blutglucosegehalt und je nach Rezeptur der Testchemie, verändert. Bei den optischen Systemen besteht diese Veränderung in aller Regel in einer lokalen Farbänderung, die remissionsphotometrisch gemessen werden kann. Details dieses Verfahrens sind grundsätzlich aus der Literatur hinreichend bekannt.

Hierbei stellt sich grundsätzlich die Problematik, dass die Vermessung der Verfärbung der Testfelder mit nicht ortsaufgelösten Sensoren, also beispielsweise mit einer einzigen Photodiode, problembehaftet ist, da hierbei die Position der Grauverfärbung oder Verfärbung auf dem Testfeld mechanisch sehr genau und mit geringen mechanischen und/oder optischen Toleranzen erfasst werden müsste. Dies ist bei einer für den Einstich und die Blutgewinnung beweglichen Lanzette nur schwer zu bewerkstelligen. Soll beispielsweise eine Toleranz der lateralen Position von weniger als 10 % erreicht werden, so darf die Positionstoleranz bei einer Kapillare mit einer Breite von 120 µm ca. ±10 µm nicht übersteigen, was mechanisch eine erhebliche Herausforderung darstellt.

Aus diesem Grund wurde mehrfach die Verwendung eines ortsaufgelösten Detektors, beispielsweise einer CMOS-Kamera, vorgeschlagen. Beispielsweise beschreibt US 6,847,451 B1 Vorrichtungen und Verfahren zur Bestimmung der Konzentration eines Analyten in einer physiologischen Probe. Dabei werden mindestens eine Lichtquelle sowie mindestens ein Detektor-Array verwendet, sowie Mittel zur Bestimmung, ob eine ausreichende Menge an Probe auf einer Mehrzahl verschiedener Flächen vorhanden ist. Unter anderem wird dabei vorgeschlagen, als Detektor-Array ein CCD-Array zu verwenden. Alternativen zur ortsaufgelösten Detektion wären beispielsweise eine ortsaufgelöste Beleuchtung oder eine Mischung derartiger Verfahren, wie beispielsweise Verfahren, die auf einem zeilenweisen Abtasten basieren.

US 2004/0095360 A1 beschreibt eine Benutzerschnittstelle einer Bildaufnahmevorrichtung und ein Bildverarbeitungsverfahren, welches beispielsweise für die Auswertung biologischer Proben wie Schwangerschaftstests oder Drogentests verwendet werden kann. Dabei wird ein hochauflösender Kamerasensor zur tatsächlichen Bilderfassung eingesetzt, welcher als Farbsensor ausgestaltet ist. Unter anderem wird dabei die Verwendung einer Linie und einer Referenzlinie innerhalb des Tests vorgeschlagen.

US 7,344,081 B2 beschreibt ein Verfahren zur automatischen Erkennung eines Testergebnisses einer Probenzone eines Teststreifens. Dabei werden ein Bild eines Barcodes und ein Bild mindestens eines Teststreifens aufgenommen. Es wird eine Farbantwort des Teststreifens auf eine Probenaufgabe bestimmt. Um einen Barcode aufzulösen, ist es jedoch inhärent notwendig, einen Detektor mit einer hohen Auflösung und damit einer hohen Anzahl von Pixeln zu verwenden.

US 5,083,214 beschreibt eine Vorrichtung und ein Verfahren zur Bestimmung von geeigneten Probennahmepunkten. Dabei wird ein Array-Detektor über einen in Form eines Mikrofilms vorliegenden Code gescannt, wobei aus der speziellen Art der Codierung eine Reduktion der aufzunehmenden Daten erreicht wird. Eine Herausforderung bei diesem Verfahren besteht darin, sich bewegende Teile zu erkennen und dabei insbesondere Informationen in digitaler Form zu erfassen.

Aus DE 196 31 086 A1 ist eine aktive Pixelbildsensorzeile bekannt, die Schutzringe, schützende Diffusionen oder eine Kombination dieser beiden Techniken verwendet, um zu verhindern, dass am Rand eines aktiven Bereichs erzeugte Elektroden auf eine Bildsensormatrix treffen. Aus US 2007/0046803 A1 ist ein CMOS-Bildsensor mit einer Mehrzahl an aktiven Pixelzeilen und einer optisch schwarzen Pixelzeile bekannt. Die optisch schwarze Pixelzeile wird aktiviert, um ein jeweiliges optisch schwarzes Signal bei Aktivierung jeder von mindestens zwei der aktiven Pixelzeilen zu generieren. Beide Dokumente behandeln spezielle Aspekte des Chipdesigns optisch sensitiver Chips.

Sämtliche bekannten Ansätze zur Bildauswertung von Testelementen weisen jedoch das Problem auf, dass ein Bild mit vergleichsweise hoher Auflösung detektiert werden und, beispielsweise mit Verfahren der Mustererkennung, analysiert werden muss. Eine vergleichsweise hohe Auflösung bedeutet hier beispielsweise eine Anzahl von 1 Million Pixel, wobei jedoch grundsätzlich auch Pixelarrays mit einer geringeren Anzahl an Pixeln eingesetzt werden. Nichtsdestotrotz ist immer eine noch hohe Datenmenge in kurzer Zeit, beispielsweise 100 ms, an eine Peripherieelektronik zu übermitteln und von dieser on-line auszuwerten, was insbesondere in tragbaren, beispielsweise handgehaltenen, Geräten auf grund der hierfür benötigten hohen Taktraten der Elektronik und aufgrund der großen Zahl von Rechenoperationen die Lebensdauer der Batterie maßgeblich begrenzen würde. Eine Teillösung bietet sich in der Vorverarbeitung der Bildinformation in einer peripheren Elektronik an. Derartige Verfahren und Vorrichtungen werden beispielsweise in EP 1 351 189 A1, in US 2005/0013494 A1 oder in US 2003/0123087 A1 beschrieben. Alternativ bietet sich eine Vorverarbeitung in Teilen bereits direkt auf einem CMOS-Sensor an, wie beispielsweise in US 6,515,702 B1 beschrieben.

Alternativ zu einer klassischen on-chip- oder off-chip-Mustererkennung, welche das Bild zur weiteren Analyse in benetzte, d.h. Glucose-informationstragende, und unbenetzte Bereiche trennt, wurde ein Verfahren einer Histogrammauswertung vorgeschlagen. Dieses Verfahren ist in EP 1 843 148 A1 beschrieben. Dabei wird für die detektierten Lichtintensitäten eine Häufigkeitsverteilung ermittelt, wobei die Häufigkeitsverteilung mindestens ein erstes Maximum, hervorgerufen durch unbenetzte Teilbereiche, und ein zweites Maximum, hervorgerufen durch benetzte Teilbereiche, aufweist. Aus der Häufigkeitsverteilung wird die Konzentration des Analyten ermittelt. Wenngleich die beispielsweise direkt auf dem CMOS-Bildsensor implementierte Histogrammanalyse die zu übermittelnde und auszuwertende Datenmenge deutlich reduzieren würde, erfordert das vorgeschlagene Verfahren allerdings, wenn eine vorausgehende Bildvorverarbeitung vermieden werden soll, realistischerweise immer noch deutlich über 10000 Pixel, um auf diese Weise eine hinreichend genaue Glucosemessung zu ermöglichen.

Zu den bisherigen Analysen und Vorschlägen kommt nunmehr aus Sicht der Messtechnik die Anforderung hinzu, dass die Abmessungen typischer Messgeräte sehr klein zu halten sind, was in der Flexibilität des Optiklayouts zu deutlichen Konsequenzen führt. Mit zunehmender Verkleinerung muss die für die Abbildung eines Testflecks auf einen Detektor verwendete Linse zunehmend höhere Brechkraft aufweisen, was zu zunehmenden Bildfehlern führt. Beispielsweise wird hierdurch die Abbildung an den Rändern unscharf. Um die Bildqualität nicht weiter zu gefährden, sind zudem aus Sicht der Optik möglichst kleine Pixel wünschenswert. Gleichzeitig ist die Pixelgröße am Detektor wegen der Halbleiterprozesstechnik derartiger Sensoren in der Regel begrenzt auf Werte von mindestens 4 µm, vorzugsweise mehr als 8 µm und besonders bevorzugt mehr als 20 µm. Dies bedeutet, dass die Halbleitertechnik in der Regel möglichst große Pixel erfordert, das Optiklayout hingegen möglichst kleine. Dies führt umgekehrt wiederum zur Notwendigkeit einer vergrößernden Abbildung und damit in praxisnahen, kostengünstigen Systemen nochmals zu einer verschlechterten Abbildungsqualität. Ferner steigt mit zunehmendem Abbildungsmaßstab bei herkömmlichen Systemen die Anforderung an die Positionstoleranz des Testfeldes.

### Aufgabe der Erfindung

Es ist dementsprechend eine Aufgabe der vorliegenden Erfindung, eine Vorrichtung und ein Verfahren anzugeben, welche die oben beschriebenen Nachteile bekannter Vorrichtungen und Verfahren vermeiden. Insbesondere soll die Vorrichtung als tragbares Handgerät ausgestaltet werden können und soll mit einfacher Elektronik, einfacher Optik und geringem Ressourcen- und Energieverbrauch in der Lage sein, zuverlässig einen optischen Nachweis mindestens eines Analyten in einer Körperflüssigkeit mit hoher Messgenauigkeit zu führen.

### Offenbarung der Erfindung

Diese Aufgabe wird gelöst durch eine Vorrichtung und ein Verfahren mit den Merkmalen der unabhängigen Ansprüche. Vorteilhafte Weiterbildungen der Erfindung, welche einzeln oder in Kombination realisierbar sind, sind in den abhängigen Ansprüchen dargestellt.

In einem ersten Aspekt der vorliegenden Erfindung wird eine Vorrichtung zum Nachweis mindestens eines Analyten in einer Körperflüssigkeit vorgeschlagen, welche mindestens ein Testelement umfasst. Die Vorrichtung kann beispielsweise als tragbares Gerät, insbesondere als handgehaltenes Gerät oder Handgerät ausgestaltet sein und kann beispielsweise über eine interne Energiequelle, beispielsweise einen elektrischen Energiespeicher wie z.B. eine Batterie und/oder einen Akkumulator, verfügen. Die Vorrichtung kann insbesondere als tragbares Testgerät ausgestaltet sein.

Als Analyt kommen grundsätzlich beliebige Arten von nachweisbaren Analyten und/oder Parametern der Körperflüssigkeit in Betracht. Besonders bevorzugt ist es, wenn der Analyt mindestens einen Metaboliten umfasst. Beispiele typischer nachweisbarer Analyten sind Glucose, Cholesterin, Lactat oder andere Analyten. Auch Kombinationen von Analyten können grundsätzlich nachgewiesen werden. Als Körperflüssigkeit kommen beispielsweise Blut, interstitielle Flüssigkeit, Speichel, Urin oder andere Körperflüssigkeiten zum Einsatz.

Die Vorrichtung umfasst mindestens ein Testelement. Unter einem Testelement ist allgemein ein Element, welches derart eingerichtet ist, dass dieses mindestens eine nachweisbare Veränderung durch den Analyten, beispielsweise infolge eines Kontakts mit dem Analyten, durchführt. Beispielsweise kann das Testelement zu diesem Zweck mindestens eine Testchemie umfassen, welche einen derartigen Analyt-spezifischen Nachweis durchführen kann. Beispiele derartiger nachweisbarer Veränderungen sind optisch nachweisbare Veränderungen, beispielsweise Farbänderungen, Grauwertänderungen und/oder andere optisch nachweisbare Veränderungen. Das Testelement kann beispielsweise mindestens ein Testfeld aufweisen, welches die Testchemie umfasst. Unter einem Testfeld ist dabei allgemein ein flächiges Element zu verstehen, welches die mindestens eine Testchemie umfasst. Daneben kann das Testfeld jedoch einen Schichtaufbau umfassen, wobei neben mindestens einer die Testchemie umfassenden Schicht mindestens eine weitere Schicht aufgebracht sein kann, beispielsweise eine Abtrennschicht. So kann das Testfeld beispielsweise eine Probenaufgabefläche umfassen, welche beispielsweise eine Oberfläche des Testfeldes sein kann. Als oberste Schicht kann beispielsweise eine Abtrennschicht vorgesehen sein, welche störende Bestandteile der Probe abtrennen kann, beispielsweise rote Blutkörperchen. Weiterhin kann das Testfeld mindestens eine Nachweisschicht umfassen, welche ihrerseits die Testchemie umfasst und welche vorzugsweise unterhalb der optionalen Abtrennschicht vorgesehen sein kann. Weiterhin kann das Testfeld eine Detektionsseite aufweisen, von welcher hier die nachweisbare Veränderung beobachtbar ist. Beispielsweise kann die Detektionsseite der Probenaufgabeseite gegenüberliegend angeordnet sein. Beispielsweise kann der Schichtaufbau derart ausgestaltet sein, dass die störenden Bestandteile der Probe wie beispielsweise rote Blutkörperchen von der Detektionsseite her nicht mehr sichtbar sind. Von der Detektionsseite her soll hingegen die Änderung der mindestens einen Eigenschaft beobachtbar sein.

Das Testelement umfasst mindestens einen zumindest zweidimensionalen Auswertebereich. Beispielsweise kann dieser Auswertebereich auf der Detektionsseite des Testelements, beispielsweise des Testfelds, angeordnet sein. Beispielsweise kann der Auswertebereich ein Teil der Detektionsseite sein und beispielsweise auf einer der Probenaufgabeseite gegenüberliegenden Seite des Testfeldes angeordnet sein. Unter einem Auswertebereich ist allgemein ein Bereich des Testfelds zu verstehen, welcher durch die Probe der Körperflüssigkeit bei einem standardmäßig ablaufenden Test in der Vorrichtung optisch erkennbar beeinflusst wird. Beispielsweise kann dies der Bereich sein, innerhalb dessen auf der Detektionsseite beim Aufpressen einer mit der Probe gefüllten Kapillare auf die Probenaufgabeseite eine optisch sichtbare Veränderung auftritt, sei es nun durch die Probe selbst oder durch eine Analyt-spezifische Reaktion innerhalb einer Testchemie des Testelements. Der Auswertebereich wird somit definiert durch eine bestimmungsgemäße Verwendung der Vorrichtung, als derjenige Teil des Testelements, insbesondere als derjenige Teil einer Detektionsseite eines Testfelds, innerhalb dessen bei der bestimmungsgemäßen Verwendung, beispielsweise bei einem bestimmungsgemäßen Übertrag der Probe auf das Testfeld, eine Veränderung eintritt. Ausgenommen hiervon sind nicht korrekt ablaufende Übertragungsvorgänge, beispielsweise Übertragungsfehler der Probe auf das Testelement, beispielsweise Überflutungen des Testelements und/oder Unterdosierungen der Probe. Allgemein kann das Testelement beispielsweise einen Probenaufgabebereich umfassen, auf welchen bei einer bestimmungsgemäßen Verwendung der Vorrichtung Probe räumlich begrenzt übertragen wird, wobei der Probenaufgabebereich beispielsweise auf einer Probenaufgabeseite des Testfeldes angeordnet sein kann. Der Auswertebereich kann einen dem Probenaufgabebereich gegenüberliegenden Bereich umfassen, beispielsweise eine Projektion des Probenaufgabebereichs von der Probenaufgabeseite auf die Detektionsseite eines Testfelds. Beispielsweise kann der Probenaufgabebereich eine im Wesentlichen rechteckige Gestalt aufweisen, entsprechend einer äußeren Form einer Kapillaren. In diesem Fall kann auch der Auswertebereich beispielsweise im Wesentlichen rechteckig ausgestaltet sein, als Projektion des Probenaufgabebereichs von der Probenaufgabeseite auf die Detektionsseite.

Die Vorrichtung umfasst weiterhin mindestens einen ortsaufgelösten optischen Detektor. Beispielsweise kann dieser Detektor mindestens einen ortsaufgelösten optischen Sensor, beispielsweise ein Sensorarray mit einer Mehrzahl von Sensorpixeln, also optischen Einzelsensoren, umfassen. Weiterhin kann der Detektor, wie unten noch näher ausgeführt wird, eine Optik umfassen, welche eingerichtet ist, um den Auswertebereich auf den optischen Sensor, beispielsweise einen Sensorchip, abzubilden. Diese Optik kann beispielsweise eine oder mehrere Linsen und/oder andere abbildende optische Systeme umfassen.

Der Detektor weist eine Mehrzahl von Pixeln auf, beispielsweise als Bestandteile eines optischen Sensors, beispielsweise eines Sensorchips. Unter Pixeln sind dabei allgemein bildsensitive Einzelsensoren zu verstehen, welche beispielsweise in einer Matrixanordnung angeordnet sein können. Der Detektor, beispielsweise eine Optik des Detektors, ist dabei eingerichtet, um zumindest einen Teil des Testelements auf einen Bildbereich abzubilden. Unter einem Bildbereich kann dabei eine Teilmenge der Sensorpixel des Detektors, insbesondere des optischen Sensors des Detektors, verstanden werden, beispielsweise eine räumlich zusammenhängende Teilmenge von Sensorpixeln des Sensors, auf welche der Teil des Testelements abgebildet wird, so dass diese Sensorpixel Bildinformationen von dem abgebildeten Teil des Testelements empfangen. Beispielsweise kann ein Teil der Detektionsseite des Testelements, beispielsweise des Testfeldes, auf den Bildbereich abgebildet werden. Neben dem abgebildeten Teil des Testelements kann der Detektor weiterhin eingerichtet sein, um weitere Teile der Vorrichtung auf den optischen Sensor abzubilden, beispielsweise einen Teil einer Lanzette und/oder Kapillaren. Dementsprechend können weitere Bildbereiche vorgesehen sein, welche nicht Abbildungen des Testelements enthalten, sondern Abbildungen anderer Teile der Vorrichtung.

Bei der Abbildung des Testelements, welche vollständig oder zumindest teilweise erfolgen kann, beispielsweise bei einer Abbildung der Detektionsseite des Testelements bzw. des Testfeldes oder eines Teils der Detektionsseite, auf den Bildbereich, soll zumindest ein Teil des Auswertebereichs auf einen Auswertebildbereich abgebildet werden. Der Auswertebereich ist somit vorzugsweise zumindest teilweise Bestandteil des Teils des Testelements, welcher auf den Bildbereich abgebildet wird. Der Auswertebildbereich ist eine Untermenge und/oder Teilmenge und/oder ein Teil des Bildbereichs, beispielsweise eine zusammenhängende Teilmenge von Sensorpixeln des Sensors, welche bei der Abbildung des Auswertebereichs Bildinformationen des Auswertebereichs empfangen.

Dabei wird vorgeschlagen, dass der Detektor derart an das Testelement oder insgesamt an die Vorrichtung angepasst ist, dass innerhalb des Auswertebildbereichs für jede Dimension des Auswertebildbereichs eine vorgegebene Mindestzahl an Pixeln vorgesehen ist. Dies bedeutet, dass in jeder Richtung des Auswertebildbereichs, beispielsweise in einer x- und einer y-Richtung, jeweils eine Mindestzahl von Pixeln Nₓ bzw. Ny vorgesehen ist. Wie unten noch näher ausgeführt wird, kann beispielsweise der Auswertebildbereich eine Richtung y senkrecht zu einer Längserstreckung der Kapillare bzw. des Abdrucks der Kapillare bzw. des Abbilds der Kapillare auf dem Auswertebildbereich aufweisen, welcher auch als Breitendimension oder Breitseite bezeichnet wird, und eine Koordinate x parallel zu einer Längserstreckung der Kapillare bzw. des Abbilds der Kapillare bzw. des Abbilds des Abdrucks der Kapillare, welche auch als Längsrichtung oder Längsseite bezeichnet werden kann. Die Breitseite und die Längsseite können insbesondere im Wesentlichen senkrecht aufeinander stehen. Die Pixel sind in einer zweidimensionalen Matrixanordnung angeordnet. Die Matrixanordnung weist Pixelzeilen und Pixelspalten auf. Die Pixelzeilen sind im Wesentlichen parallel zu einer Längsrichtung des Auswertebereichs und/oder des Auswertebildbereichs angeordnet sind.

Der Auswertebereich kann insbesondere, wie oben dargestellt, ein Teil des Testelements sein. Insbesondere kann es sich hierbei um einen Teil eines Testfelds des Testelements mit mindestens einer Nachweischemie zum Nachweis des Analyten handeln, beispielsweise um einen Teil einer Detektionsseite des Testfelds. Die Vorrichtung kann insbesondere derart eingerichtet sein, dass zum Nachweis des Analyten Körperflüssigkeit auf einen Probenaufgabebereich des Testelements übertragen wird, beispielsweise auf eine Probenaufgabeseite eines Testfelds. Dieser Probenaufgabebereich kann beispielsweise bei einer bestimmungsgemäßen Verwendung der Vorrichtung räumlich begrenzt sein, beispielsweise indem dieser im Wesentlichen einem Abdruck einer Kapillare auf das Testfeld, beispielsweise auf die Probenaufgabeseite, entspricht, also einem Bereich, innerhalb dessen von der Kapillare Körperflüssigkeit, beispielsweise Blut, auf die Probenaufgabeseite übertragen wird.

Die Vorrichtung kann insbesondere derart eingerichtet sein, dass zum Nachweis des Analyten Körperflüssigkeit, insbesondere Blut und/oder interstitielle Flüssigkeit, auf das Testelement übertragen wird. Dieser Übertrag kann, wie oben dargestellt, auf einen räumlich begrenzten Probenaufgabebereich einer Probenaufgabeseite eines Testfelds erfolgen. Auch andere Ausgestaltungen sind jedoch grundsätzlich möglich. Der Übertrag kann beispielsweise dadurch erfolgen, dass ein Übertragungselement an das Testelement, beispielsweise an eine Probenaufgabeseite eines Testfeldes, angenähert wird. Diese Annäherung kann bis zu einem physikalischen Kontakt zwischen dem Übertragungselement und der Probenaufgabeseite eines Testfeldes führen. Beispielsweise kann das Übertragungselement, wie oben dargestellt, eine Kapillare umfassen, beispielsweise eine Kapillare innerhalb einer Lanzette. Derartige Lanzetten mit Kapillaren werden häufig auch als Microsampler bezeichnet. Der Probenaufgabebereich kann insbesondere dem Auswertebereich entsprechen, beispielsweise indem der Auswertebereich ein Bereich des Testelements ist, innerhalb dessen, wie oben beschrieben, eine Veränderung bei einem ordnungsgemäßen Übertrag von Körperflüssigkeit auf den Probenaufgabebereich auftritt, welche optisch nachweisbar ist. Beispielsweise kann der Auswertebereich ein dem Probenaufgabebereich gegenüberliegender Bereich eines Testfeldes sein, beispielsweise eine Projektion des Probenaufgabebereichs von der Probenaufgabeseite auf die Detektionsseite, sofern beispielsweise laterale Ausbreitungseffekte beim Durchdringen des Testfelds vernachlässigt werden können. Auf diese Weise kann beispielsweise ein Übertrag der Körperflüssigkeit auf den Probenaufgabebereich auf der Probenaufgabeseite eines Testfelds erfolgen, wohingegen eine Detektion von der Rückseite her, also von der Detektionsseite her erfolgt, wo innerhalb des Auswertebereichs eine optisch nachweisbare Veränderung detektierbar sein kann.

Wie oben dargestellt, kann die Vorrichtung insbesondere mindestens ein Lanzettenelement mit mindestens einer Kapillare umfassen. Beispielsweise kann die Vorrichtung eine Antriebsvorrichtung umfassen, mittels derer eine Stechbewegung des Lanzettenelements antreibbar ist, beispielsweise umfassend eine Vorwärtsbewegung (Einstichbewegung) und eine Rückbewegung. Während des Einstichvorgangs und/oder während der Rückbewegung kann in der Kapillaren Körperflüssigkeit aufgenommen werden. Die Vorrichtung kann dann insbesondere eingerichtet sein, um mittels der Kapillaren Körperflüssigkeit aufzunehmen und durch Annäherung der Kapillare an das Testelement Körperflüssigkeit auf das Testelement, insbesondere auf ein Testfeld mit mindestens einer Nachweischemie, zu übertragen. Beispielsweise kann dieser Übertrag auf einen Probenaufgabebereich einer Probenaufgabeseite des Testelements, insbesondere des Testfelds, erfolgen. Die Annäherung der Kapillaren an das Testelement, beispielsweise an die Probenaufgabeseite des Testfelds, kann insbesondere mittels mindestens eines Aktors erfolgen. So kann beispielsweise ein Aktor vorgesehen sein, welcher die zumindest teilweise mit der Körperflüssigkeit gefüllte Kapillare an das Testfeld, beispielsweise an die Probenaufgabeseite, annähert, bis der Übertrag stattfindet. Beispielsweise kann ein Aufpressen der Kapillare auf die Probenaufgabeseite des Testfelds erfolgen. Auch eine kontaktfreie Annäherung ist jedoch grundsätzlich möglich, beispielsweise eine Annäherung auf einen derart kurzen Abstand, dass ein Probenübertrag von der Kapillare auf die Probenaufgabeseite beispielsweise durch Kapillarkräfte zwischen der Lanzette und dem Testfeld und/oder Adhäsionskräfte erfolgt. Alternativ oder zusätzlich zu einem Aktor kann die Vorrichtung jedoch auch auf andere Weise eingerichtet sein, um die Annäherung der Kapillare an das Testelement zu bewirken. Beispielsweise kann bei einer Rückholbewegung der Lanzette die Lanzette bzw. die Kapillare derart geführt werden, dass diese eine räumliche Bahn beschreibt, innerhalb derer die Annäherung an das Testelement, beispielsweise an die Probenaufgabeseite des Testfelds, erfolgt. Beispielsweise kann eine gekrümmte Führung der Lanzette vorgesehen sein, innerhalb derer die Lanzette eine gekrümmte Bahn beschreibt, mittels derer die Lanzette bzw. die Kapillare auf das Testfeld aufgepresst und/oder an dieses angenähert wird. Verschiedene andere Ausgestaltungen oder Kombinationen der genannten und/oder anderer Ausgestaltungen zur Annäherung der Kapillare an das Testelement sind möglich.

Wie oben beschrieben, kann der Auswertebereich insbesondere ein Bereich des Testelements sein, in welchem durch den Übertrag der Körperflüssigkeit auf das Testelement eine optisch nachweisbare Veränderung auftritt. Diese Veränderung kann durch die Körperflüssigkeit selbst hervorgerufen werden, oder, in mehr oder minder starkem Ausmaß, durch den in der Körperflüssigkeit enthaltenen mindestens einen Analyten und beispielsweise dessen Reaktion mit der mindestens einen Testchemie. Wie oben beschrieben, kann der Auswertebereich somit insbesondere Teil eines Testfelds sein, beispielsweise Teil einer Detektionsseite eines Testfelds, welche beispielsweise auch einer Probenaufgabeseite des Testfelds gegenüberliegen kann, beispielsweise indem der Auswertebereich einer Projektion eines Probenaufgabebereichs der Probenaufgabeseite bei einer bestimmungsgemäßen Verwendung der Vorrichtung entspricht. Beispielsweise kann der Auswertebereich ein Bereich sein, in welchem durch die Probe eine optisch nachweisbare Veränderung, beispielsweise eine Farbänderung und/oder eine Grauwertveränderung, auftritt. Insbesondere kann der Auswertebereich ein Abbild der Kapillare von der Probenaufgabeseite auf die Detektionsseite sein, oder ein Teilbereich dieser Projektion.

Die Kapillare kann insbesondere eine Breite von 50 bis 200 µm, insbesondere von 90 bis 150 µm und besonders bevorzugt von ca. 120 µm aufweisen. Alternativ oder zusätzlich kann die Kapillare insbesondere eine Länge von mindestens 1 mm, insbesondere mindestens 2 mm und vorzugsweise eine Länge von 2 bis 4 mm aufweisen. Typischerweise weisen Kapillaren eine Tiefe von 20 bis 150 µm auf, beispielsweise eine Tiefe von 50 bis 120 µm. Auch andere Dimensionen der Kapillare sind jedoch grundsätzlich möglich.

Die Vorrichtung kann insbesondere eingerichtet sein, um den Auswertebereich automatisch zu erkennen. Zu diesem Zweck kann die Vorrichtung beispielsweise eine Auswertevorrichtung aufweisen, welche beispielsweise ganz oder teilweise in den Detektor integriert sein kann, welche jedoch auch ganz oder teilweise extern angeordnet sein kann. Diese Auswertevorrichtung kann beispielsweise eine oder mehrere Datenverarbeitungsvorrichtungen umfassen. Alternativ oder zusätzlich kann die Auswertevorrichtung jedoch auch auf einfachere Weise ausgestaltet sein und kann beispielsweise einen oder mehrere Komparatoren umfassen und/oder andere elektronische Vorrichtungen, um Signale des Detektors, beispielsweise Signale des optischen Sensors und/oder eines oder mehrerer Pixel des optischen Sensors, mit einem oder mehreren Schwellwerten zu vergleichen. Alternativ oder zusätzlich zur Aufgabe der Erkennung des Auswertebereichs kann die Auswertevorrichtung auch andere Aufgaben haben, beispielsweise die Aufgaben, eine Datenreduktion durchzuführen, die Aufgabe, nicht-bestimmungsgemäße Arbeitsabläufe zu erkennen, die Aufgabe, eine Vorverarbeitung von Bilddaten vorzunehmen oder ähnliche Aufgaben.

Bei einer automatischen Erkennung des Auswertebereichs können verschiedene Verfahren verwendet werden. In einer ersten Verfahrensvariante wird ausgenutzt, dass der Auswertebereich vorzugsweise eine Projektion einer Kapillare auf die Detektionsseite des Testelements darstellen kann. Dementsprechend kann bei dieser ersten Verfahrensvariante ein Mustererkennungsverfahren eingesetzt werden, bei welchem eine Lanzette und/oder eine Kapillare der Vorrichtung erkannt wird. So kann beispielsweise die Vorrichtung derart eingerichtet sein, dass die Kapillare über das Testelement hinausragt, so dass mittels des Detektors nicht nur ein Bild der Detektionsseite des Testelements aufgenommen wird, sondern auch ein Teilbereich der Lanzette und/oder der Kapillare, in welchem diese nicht auf der Probenaufgabeseite des Testelements aufliegt. Beispielsweise kann, wie oben dargestellt, ein Testfeld vorgesehen sein, mit einer Probenaufgabeseite, an welche die Lanzette mit der Kapillare angenähert wird, und einer gegenüberliegenden Detektionsseite, welche von dem Detektor betrachtet wird. Ragt die Lanzette mit der Kapillare seitlich über das Testfeld hinaus, so nimmt der Detektor vorzugsweise einen Teil der Lanzette und der Kapillare auf, welcher nicht durch das Testfeld optisch verdeckt wird. Das Mustererkennungsverfahren kann insbesondere derart ausgestaltet sein, dass als Auswertebereich eine Extrapolation der Lanzette und/oder Kapillare auf das Testelement identifiziert wird. Beispiele dieser ersten Verfahrensvariante werden unten noch näher erläutert.

In einer zweiten, alternativ oder zusätzlich einsetzbaren Variante des Verfahrens bzw. der Vorrichtung kann ein Signaländerungsverfahren eingesetzt werden. Bei einem Signaländerungsverfahren werden Änderungen der Signale des optischen Sensors des Detektors überwacht. Dabei wird als Auswertebereich ein Bereich des Testelements identifiziert, innerhalb dessen durch einen Übertrag der Körperflüssigkeit auf das Testelement eine optisch nachweisbare Veränderung eintritt.. Wie oben dargestellt, kann es sich bei dieser optisch nachweisbaren Veränderung um eine Veränderung handeln, welche durch die Körperflüssigkeit selbst bewirkt wird, beispielsweise indem die Körperflüssigkeit selbst zu einer Verdunklung und/oder Grauwertänderung und/oder Farbänderung innerhalb des Auswertebereichs auf der Detektionsseite des Testelements, beispielsweise des Testfeldes, führt. Alternativ oder zusätzlich können diese optischen Änderungen jedoch auch durch den nachzuweisenden Analyten selbst hervorgerufen werden. In beiden Fällen lässt sich mittels des Signaländerungsverfahrens die Lage des Auswertebereichs bestimmen. Beispielsweise kann ein Auswertebereich durch eine Erkennung von durch den Kapillarrand bedingten Inhomogenitäten in der optisch nachweisbaren Veränderung, beispielsweise in einer Verfärbung und/oder Verdunklung und/oder Grauwertänderung, erkannt und definiert werden.

Dieser Aspekt der vorgeschlagenen Erfindung kann auch unabhängig von den übrigen Aspekten der Vorrichtung realisiert werden. So wird in einem nebengeordneten Aspekt ein Verfahren zur Erkennung eines Auswertebereichs eines Testelements vorgeschlagen, insbesondere unter Verwendung einer oben oder im Folgenden beschriebenen Vorrichtung. Auch die Verwendung anderer Arten von Vorrichtungen ist jedoch grundsätzlich denkbar. Allgemein wird bei dem Verfahren mindestens ein Lanzettenelement, beispielsweise gemäß der obigen Beschreibung, mit mindestens einer Kapillaren verwendet. In der Kapillare aufgenommene Körperflüssigkeit wird auf das Testelement übertragen, beispielsweise auf eine Probenaufgabeseite eines Testfelds des Testelements. Weiterhin wird mittels mindestens eines ortsaufgelösten optischen Detektors, beispielsweise gemäß der obigen Beschreibung, zumindest ein Teil des Testelements, beispielsweise ein Teil einer Detektionsseite eines Testfelds des Testelements, auf einen Bildbereich abgebildet, beispielsweise einen Bildbereich eines optischen Sensors des Detektors. Dabei wird zumindest ein Teil des Auswertebereichs des Testelements auf einen Auswertebildbereich abgebildet. Das Verfahren wird derart durchgeführt, dass der Auswertebereich automatisch erkannt wird, nach einem Verfahren ausgewählt aus: einem Mustererkennungsverfahren, wobei bei dem Mustererkennungsverfahren die Lanzette und/oder die Kapillare erkannt wird, wobei als Auswertebereich eine Extrapolation der Lanzette und/oder der Kapillaren auf das Testelement identifiziert wird; ein Signaländerungsverfahren, wobei als Auswertebereich ein Bereich des Testelements identifiziert wird, innerhalb dessen durch den Übertrag der Körperflüssigkeit auf das Testelement eine optisch nachweisbare Veränderung eintritt. Letztere Verfahrensvariante kann beispielsweise mittels einfacher Vergleichsverfahren durchgeführt werden, beispielsweise indem ein Pixel, mehrere Pixel oder alle Pixel eines optischen Sensors des Detektors überwacht werden, die Signale dieser Pixel mit zuvor aufgenommenen Signalen verglichen werden und beispielsweise die Signaländerungen mit Schwellwerten verglichen werden. Überschreiten die Signaländerungen vorgegebene Schwellwerte, so kann beispielsweise darauf geschlossen werden, dass eine Benetzung stattgefunden hat und dass die zugehörigen Pixel innerhalb des Auswertebereichs bzw. innerhalb des Auswertebildbereichs angeordnet sind. Für weitere mögliche Ausgestaltungen kann auf die obige Beschreibung verwiesen werden.

Der Detektor kann insbesondere derart eingerichtet sein, dass dieser bzw. ein optischer Sensor dieses Detektors eine Gesamtzahl von maximal 1000 Pixeln aufweist, vorzugsweise eine Gesamtzahl von maximal 500 und besonders bevorzugt eine Gesamtzahl von maximal 256 Pixeln. Beispielsweise können Detektoren eingesetzt werden, welche eine Längsseite und eine Breitseite aufweisen. Wie oben definiert, kann die Längsseite insbesondere als x-Richtung definiert sein, welche im Normalfall, also bei einer bestimmungsgemäßen Verwendung der Vorrichtung, parallel zur Kapillaren bzw. parallel zum Abbild der Kapillaren im Bildbereich angeordnet ist. Die Breitseite kann dementsprechend senkrecht zur Kapillaren bzw. senkrecht zum Abbild der Kapillaren ausgerichtet sein und kann beispielsweise als y-Richtung definiert werden. Der Detektor kann insbesondere derart ausgestaltet sein, dass dieser in Richtung der Breitseite mindestens 3 Pixel, vorzugsweise maximal 100 Pixel, insbesondere 20 bis 50 Pixel und besonders bevorzugt 32 Pixel aufweist. Der Detektor kann weiterhin in der Richtung der Längsseite, also in x-Richtung, mindestens 1 Pixel, vorzugsweise 2 bis 20 Pixel, insbesondere 5 bis 10 Pixel und besonders bevorzugt 7 Pixel aufweisen. Auch andere Ausgestaltungen sind jedoch grundsätzlich möglich. Besonders bevorzugt ist es, wenn der Detektor derart ausgestaltet ist, dass innerhalb des Auswertebereichs, also innerhalb des Bereichs, in dem sich bei einer ordnungsgemäßen Verwendung der Vorrichtung und beispielsweise bei einem ordnungsgemäßen Übertrag von Körperflüssigkeit auf das Testelement optisch nachweisbare Veränderungen bemerkbar machen, mindestens 3 Pixel angeordnet sind, insbesondere 5 bis 30 Pixel und besonders bevorzugt 10 Pixel.

Der Auswertebereich kann insbesondere, wie oben ausgeführt, eine Längsseite und eine Breitseite aufweisen, insbesondere eine parallel zur Kapillaren bzw. des Abbilds der Kapillaren auf dem Bildbereich ausgerichtete Längsseite und eine senkrecht zur Kapillaren bzw. deren Abbild ausgerichtete Breitseite. Die Breitseite kann, wie oben ausgeführt, als y-Richtung definiert werden, und die Längsseite als x-Richtung, wobei diese Richtungen vorzugsweise im Wesentlichen senkrecht aufeinander stehen, beispielsweise mit einer Abweichung von nicht mehr als 5°. Pixel gleicher y-Koordinate können dann als Pixelzeile bezeichnet werden, und Pixel gleicher x-Koordinate als Pixelspalte. Der Detektor kann insbesondere derart ausgestaltet sein, dass innerhalb des Auswertebereichs in Richtung der Breitseite mindestens 3 Pixelzeilen angeordnet sind, insbesondere 3 bis 10 Pixelzeilen. Alternativ oder zusätzlich kann der Detektor derart ausgestaltet sein, dass in Richtung der Längsseite mindestens eine Pixelspalte, vorzugsweise mindestens drei Pixelspalten, insbesondere 3 bis 10 Pixelspalten und besonders bevorzugt 7 Pixelspalten angeordnet sind.

Die Pixel können insbesondere eine langgestreckte Pixelgeometrie aufweisen. Unter einer langgestreckten Pixelgeometrie ist dabei eine Geometrie zu verstehen, bei welcher die Pixel in einer Dimension eine größere Ausdehnung aufweisen als in einer anderen Dimension. Beispielsweise können die Pixel in x-Richtung eine höhere Länge aufweisen als in y-Richtung. So kann beispielsweise der Auswertebereich eine Längsseite und eine Breitseite aufweisen, insbesondere eine parallel zur Kapillaren ausgerichtete Längsseite und eine senkrecht zur Kapillaren ausgerichtete Breitseite. Die Pixel können insbesondere in Richtung der Längsrichtung, also beispielsweise in x-Richtung, eine Länge aufweisen und in Richtung der Breitseite, vorzugsweise in y-Richtung, eine Breite. Die Länge kann dabei die Breite vorzugsweise überschreiten. Insbesondere kann die Länge die Breite um mindestens einen Faktor 1,3, insbesondere um mindestens einen Faktor 1,7 oder mindestens einen Faktor 2 und besonders bevorzugt um einen Faktor 2,3 überschreiten. Derartige Pixelgeometrien haben sich in der Praxis für langgestreckte Kapillaren mit typischen Dimensionen, beispielsweise die oben dargestellten Kapillardimensionen, als besonders geeignet erwiesen, um zuverlässig den Auswertebereich zu erfassen und die optisch nachweisbaren Änderungen auszuwerten. Die Länge der Pixel kann beispielsweise 10 bis 300 µm betragen, vorzugsweise 50 bis 100 µm und besonders bevorzugt 70 µm. Die Breite kann beispielsweise 5 bis 200 µm betragen, vorzugsweise 10 bis 100 µm und besonders bevorzugt 30 µm.

Die Pixel sind in einer zweidimensionalen Matrixanordnung angeordnet sein. Die Matrixanordnung weist Pixelzeilen und Pixelspalten auf, beispielsweise wie oben beschrieben. So können beispielsweise die Zeilen parallel zur x-Richtung ausgerichtet sein, und die Pixelspalten parallel zur y-Richtung. Die Pixelzeilen sind im Wesentlichen parallel zur Längsrichtung des Auswertebereichs angeordnet, beispielsweise im Wesentlichen parallel zu einer Abbildung einer Längserstreckungsachse der Kapillaren bzw. des Abbilds der Kapillaren in dem Bildbereich. Unter "im Wesentlichen parallel" kann dabei insbesondere eine Abweichung von einer vollständigen Parallelität von weniger als 5°, insbesondere eine Abweichung von weniger als 2° und besonders bevorzugt eine Abweichung von 1° oder weniger, insbesondere 0°, verstanden werden. Die Längsrichtung des Auswertebereichs, also beispielsweise eine Längserstreckungsachse der Kapillaren und/oder eines Abbilds der Kapillaren in dem Bildbereich, kann also im Wesentlichen parallel zu den Pixelzeilen angeordnet sein. Diese Ausgestaltung der Vorrichtung, insbesondere in Kombination mit den oben beschriebenen länglichen Pixeln, führt zu einer besonders effizienten Auswertung des Auswertebereichs, bei möglichst geringer Pixelzahl, der Möglichkeit einer Verwendung großer Pixelflächen und dennoch einer zuverlässigen Auswertung einer Vielzahl von Pixeln innerhalb des Auswertebereichs.

Der Detektor kann insbesondere, wie oben bereits ausgeführt, eine ortsauflösende Optik aufweisen. Diese ortsauflösende Optik kann beispielsweise eine oder mehrere Linsen auf weisen und/oder andere optische abbildende Systeme. Weiterhin kann die ortsauflösende Optik weitere optische Elemente mit nicht-abbildenden Eigenschaften aufweisen, beispielsweise Blenden oder ähnliches. Weiterhin können beispielsweise Filter, Spiegel, andere Arten von optischen Umlenkelementen oder andere optische Elemente vorgesehen sein.

Die ortsauflösende Optik kann insbesondere eingerichtet sein, um den Auswertebereich mit einer Vergrößerung von 3:1 bis 0,5:1 auf den Auswertebildbereich abzubilden, vorzugsweise mit einer Vergrößerung von 2:1 bis 0,8:1, besonders bevorzugt mit einer Vergrößerung von 1,1:1 bis 0,9:1 und idealerweise von 1:1. Eine Vergrößerung von 3:1 bedeutet dabei, dass der Auswertebildbereich um einen Faktor 3 größer ist als der Auswertebereich. Idealerweise ist die Optik also derart eingerichtet, dass diese keine Vergrößerung im eigentlichen Sinne aufweist, sondern die Größe des Auswertebildbereichs im Wesentlichen der Größe des Auswertebereichs entspricht.

Wie oben beschrieben, basiert die Bestimmung des Auswertebereichs auf einer ordnungsgemäßen Benetzung. Beispielsweise kann der Auswertebereich einen Abdruck der Kapillaren bzw. eine Projektion der Kapillaren auf die Detektionsseite umfassen. Abgesehen von einer ordnungsgemäßen Benetzung, bei welcher lediglich, abgesehen von in der Regel nicht zu vermeidenden Inhomogenitäten im Randbereich der Kapillaren, Körperflüssigkeit aus der Kapillaren auf das Testelement, beispielsweise auf die Probenaufgabeseite, übertragen wird, können verschiedene Übertragungsfehler und/oder Benetzungsfehler auftreten. So kann beispielsweise die Kapillare unzureichend befüllt sein, so dass eine zu geringe Menge an Körperflüssigkeit auf die Probenaufgabeseite übertragen wird. Dieser Fall der unvollständigen Befüllung und/oder des unvollständigen Übertrags von Körperflüssigkeit auf das Testelement stellt jedoch nur einen von mehreren Fehlerfällen dar. Dieser Fall kann beispielsweise dann auftreten, wenn eine ungeeignete Einstichstelle in ein Körpergewebe gewählt worden ist, so dass beispielsweise bei einem Einstichvorgang und/oder Probennahmevorgang eine zu geringe Menge an Körperflüssigkeit von dem Microsampler aufgenommen worden ist. Auch der entgegengesetzte Fall kann auftreten. In diesem Fall kann beispielsweise die gesamte Lanzette mit Körperflüssigkeit oder Blut benetzt sein, welches dann auf das Testelement übertragen wird, so dass beispielsweise eine Überflutung des Testelements mit Körperflüssigkeit erfolgt. Auch dieser Fall kann zu Fehlern führen, beispielsweise indem, wie unten noch näher ausgeführt wird, keine nicht-benetzten Bereiche innerhalb des Bildbereichs zur Verfügung stehen, also Bereiche außerhalb des Auswertebereichs, welche als Referenzwert und/oder "Leerwert" dienen könnten, um die Verfärbung bzw. die optische Veränderung des Testelements zu charakterisieren.

Besonders bevorzugt ist es daher, wenn die Vorrichtung eingerichtet ist, um eine Benetzung des Testelements mit der Körperflüssigkeit zu charakterisieren, insbesondere zu bewerten. Diese Charakterisierung kann beispielsweise dadurch erfolgen, dass eine Auswertevorrichtung vorgesehen ist, welche Signale des optischen Sensors des Detektors auswertet, beispielsweise eine Auswertevorrichtung mit den oben beschriebenen Merkmalen. Die Auswertevorrichtung kann beispielsweise eine Charakterisierung der Benetzung derart vornehmen, dass eine ordnungsgemäße Benetzung, also ein ordnungsgemäßer Übertrag von Körperflüssigkeit auf das Testelement, unterschieden wird von einem oder mehreren Fehlerfällen. Beispielsweise kann ein ordnungsgemäßer, erfolgreicher Übertrag von Körperflüssigkeit auf das Testelement unterschieden werden von einem Fall einer Überflutung, bei welchem auch außerhalb der Grenzen der Kapillare Körperflüssigkeit auf die Probenaufgabeseite des Testelements übertragen wird, und einem Fall einer Unterdosierung, bei welchem auch innerhalb des eigentlichen Auswertebereichs eine unvollständige Benetzung der Probenaufgabeseite mit Körperflüssigkeit erfolgt. Die Charakterisierung kann insbesondere derart vorgenommen werden, dass die Vorrichtung eingerichtet ist, um mehrere Pixel in mindestens einer Dimension miteinander zu vergleichen. Beispielsweise können benachbarte Pixel in mindestens einer Richtung, beispielsweise einer Richtung parallel zu einer Längsseite des Auswertebereichs, miteinander verglichen werden. Insbesondere kann ein Vergleich zweier oder mehrerer benachbarter Pixel einer parallel zum Auswerteberteich ausgerichteten Pixelzeile erfolgen. Insbesondere können die Signale der Pixel innerhalb des Auswertebereichs verglichen werden, um zu erkennen, ob Pixel, die eigentlich eine Benetzung indizieren sollten, tatsächlich eine derartige Benetzung anzeigen. Auf diese Weise kann beispielsweise eine Unterdosierung erkannt werden, beispielsweise durch eine unvollständige Füllung der Kapillaren und/oder einen unvollständigen Übertrag der Körperflüssigkeit auf das Testelement. Andererseits kann erkannt werden, wenn Pixel, die eigentlich keine Benetzung indizieren sollten, also Pixel außerhalb des Auswertebereichs, tatsächlich doch eine Benetzung detektieren, wodurch beispielsweise eine Überflutung und/oder Überdosierung erkannt werden kann. Die Charakterisierung kann beispielsweise derart erfolgen, dass ein Vergleich benachbarter Pixel aus einer Pixelzeile erfolgt, die im Wesentlichen parallel zur Längsrichtung des Auswertebereiches angeordnet ist, wobei beispielsweise ein Schwellwertverfahren verwendet werden kann. So kann beispielsweise die Differenz der Signale der benachbarten Pixel gebildet werden und mit mindestens einem Schwellwert verglichen werden. Überschreitet die Differenz den mindestens einen Schwellwert, so kann beispielsweise auf das Vorliegen einer Unterbenetzung und/oder Unterdosierung und/oder einen anderen Fehler geschlossen werden. Hierbei ist die Längsrichtung des Auswertebereiches bevorzugt im Wesentlichen parallel zu den Rändern der Kapillare und/oder des Kapillarkanals der Kapillare ausgerichtet. Die Kapillare wird, wie oben ausgeführt, vorzugsweise zum Übertrag der Körperflüssigkeit an das Testelement angenähert, beispielsweise auf dieses aufgedrückt. Aufgrund eines Vergleichs der benachbarten Pixel der hierzu parallel ausgerichteten Pixelzeile ist es somit möglich, eine fehlerhaft befüllte Kapillare und/oder einen fehlerhaften Transfer der Körperflüssigkeit, beispielsweise aufgrund einer unvollständigen und/oder lückenhaften Befüllung der Kapillaren, zu erkennen.

Wie oben dargestellt, kann der Detektor insbesondere als kompakter Detektor ausgestaltet sein. So kann der Detektor insbesondere eine Detektorbaugruppe aufweisen, insbesondere einen Detektorchip, wobei beispielsweise die Auswertevorrichtung vollständig oder teilweise in die Detektorbaugruppe, insbesondere den Detektorchip, integriert sein kann. Die Auswertevorrichtung kann eingerichtet sein, um eine Bildauswertung des Bildbereichs und/oder des Auswertebildbereichs vollständig oder teilweise durchzuführen. Der Detektorchip kann insbesondere als anwendungsspezifischer integrierter Schaltkreis (ASIC) ausgestaltet sein.

Die Vorrichtung kann insbesondere eingerichtet sein, um einen Leerwert zu erkennen. Ein Leerwert charakterisiert dabei eine optische Eigenschaft des Bildbereichs und/oder des Auswertebildbereichs ohne Benetzung des Testelements mit Körperflüssigkeit. Die Erkennung des Leerwerts kann insbesondere wiederum unter Verwendung einer Auswertevorrichtung erfolgen, welche ganz oder teilweise in den Detektor integriert sein kann. Die Vorrichtung kann insbesondere eingerichtet sein, um den Leerwert nach einem oder mehreren der im Folgenden beschriebenen Verfahren zu bestimmen.

In einer ersten Variante eines Verfahrens, zu dessen Durchführung die Vorrichtung eingerichtet sein kann, insbesondere die Auswertevorrichtung, kann eine Aufnahme einer zeitlichen Bildsequenz erfolgen. Unter einer zeitlichen Bildsequenz ist dabei eine Mehrzahl von Bildinformationen des optischen Sensors zu verstehen, welche zu verschiedenen, aufeinander folgenden Zeiten aufgenommen wurden, beispielsweise Bilder, welche mit einem Abstand von 100 ms aufgenommen werden. Der Auswertebereich kann aus dieser zeitlichen Bildsequenz bestimmt werden, beispielsweise mittels eines oder mehrerer der oben beschriebenen Verfahren. Dabei können mindestens ein, vorzugsweise mehrere innerhalb des Auswertebereichs angeordnete Pixel erkannt werden und aus der zeitlichen Bildsequenz mindestens ein Anfangswert des Pixels bestimmt und als Leerwert verwendet werden. In anderen Worten kann zunächst aus der zeitlichen Bildsequenz der Auswertebereich ermittelt werden und dann für einen oder mehrere Pixel innerhalb des Auswertebereichs aus der aufgenommenen Bildsequenz ein oder mehrere Anfangswerte bestimmt werden, die dann als Leerwert dienen können, was einem "Zurückspulen" des Films der Bildsequenz entspricht. Ein Vorteil dieses Verfahrens liegt darin, dass ein Leerwert für jeden auszuwertenden Pixel bestimmt werden kann, der genau diesem Pixel entspricht.

Alternativ oder zusätzlich kann ein Verfahren verwendet werden, bei welchem ein Anfangswert sämtlicher Pixel des Bildbereichs gespeichert wird, oder zumindest ein Anfangswert mehrerer Pixel des Bildbereichs. Aus einer zeitlichen Bildsequenz der Pixel kann dann der Auswertebereich ermittelt werden. Pixel außerhalb des Auswertebereichs können verworfen werden, so dass auf diese Weise eine Datenreduktion erfolgen kann. Mindestens ein Anfangswert eines Pixels innerhalb des Auswertebereichs kann dann als Leerwert verwendet werden. Diese Verfahrensvariante bietet den Vorteil einer erheblichen Datenreduktion, da bereits bei der Aufnahme der zeitlichen Bildsequenz, sobald klar ist, wo der Auswertebereich innerhalb des Bildbereichs positioniert ist, Pixel außerhalb des Auswertebereichs verworfen werden können, so dass keine Bildsequenzen des gesamten Bildbereichs mehr gespeichert werden müssen, sondern lediglich Bildsequenzen der Pixel des Auswertebereichs.

In einem dritten, wiederum alternativ oder zusätzlich einsetzbaren Verfahren kann der Auswertebereich ermittelt werden, beispielsweise mittels eines oder mehrerer der oben beschriebenen Verfahren. Als Leerwert kann dann mindestens ein Pixel außerhalb des Auswertebereichs, also ein Pixel, auf welchen ein Bereich des Testelements außerhalb des Auswertebereichs abgebildet wird, als Leerwert eingesetzt werden. Diese Verfahrensvariante bietet den Vorteil, dass lediglich eine geringe Anzahl an Daten gespeichert werden muss. Die Bestimmung des Leerwerts kann beispielsweise einzig und allein anhand eines Bilds nach der Reaktion des Analyten erfolgen, ohne dass eine Historie bzw. eine zeitliche Bildsequenz abgespeichert werden muss. Auch andere Verfahren zur Ermittlung eines oder mehrerer Leerwerte sind jedoch grundsätzlich einsetzbar. Die vorgeschlagene Vorrichtung und das vorgeschlagene Verfahren weisen gegenüber bekannten Vorrichtungen und Verfahren eine Vielzahl von Vorteilen auf. So wird erfindungsgemäß insbesondere eine vorteilhafte Alternative zur Verwendung üblicher Bildsensoren mit mehr als 10000 Pixeln angeboten, wie sie beispielsweise für die Histogrammanalyse nach der EP 1 843 148 A1 verwendet werden können. Die Erfindung basiert insbesondere auf der Erkenntnis, dass zum einen eine Ortsauflösung vorzugsweise mit mindestens ca. 10 Pixeln pro Kapillarbreite, umgerechnet auf das Bild der Kapillaren auf dem Bildbereich, angestrebt ist. Zum anderen basiert die Erfindung jedoch auf der Erkenntnis, dass die optische Abbildungsqualität in kleinen, hochintegrierten und kostensensitiven Geräten, insbesondere Handgeräten, unter dem Raummangel stark leidet. Gleichzeitig wird die Erfindung der Tatsache gerecht, dass halbleitertechnisch möglichst große Pixel günstig sind, da optische Sensoren mit möglichst großen Pixeln, beispielsweise Pixeln der oben beschriebenen Pixelgeometrie und/oder Pixelgröße, einen vergleichsweise hohen Füllfaktor der optischen Sensoren ermöglichen.

So lässt sich erfindungsgemäß insbesondere eine Optik mit einer 1:1-Abbildung einsetzen. Dabei lässt sich unter Verwendung der oben beschriebenen Vorrichtung in einer oder mehreren der beschriebenen Varianten insbesondere die Fläche pro Pixel vergrößern und entsprechend die Pixelzahl reduzieren. Mit einer Reduktion der Pixelzahl, beispielsweise auf die oben beschriebenen Pixelzahlen des optischen Sensors, geht eine Verringerung der Datenmenge und des Aufwands zur Datenanalyse einher, so dass an allen der oben beschriebenen kritischen Randbedingungen der Vorrichtung eine Verbesserung erzielt werden kann. Gleichzeitig kann die Pixelgeometrie der Nachweismethode und der Realisierung angepasst werden, beispielsweise durch eine rechteckige Ausgestaltung der Pixel, wobei die Pixelgeometrie insbesondere speziell der Geometrie des Auswertebereichs, beispielsweise des Messflecks, z.B. bedingt durch die Kapillargeometrie, angepasst werden kann.

Zusammenfassend werden im Rahmen der vorliegenden Erfindung folgende Ausführungsformen als besonders vorteilhaft angesehen:
Ausführungsform 1: Vorrichtung zum Nachweis mindestens eines Analyten in einer Körperflüssigkeit, umfassend mindestens ein Testelement mit mindestens einem zweidimensionalen Auswertebereich, weiterhin umfassend mindestens einen ortsauflösenden optischen Detektor mit einer Mehrzahl von Pixeln, wobei der Detektor eingerichtet ist, um zumindest einen Teil des Testelements auf einen Bildbereich abzubilden, wobei zumindest ein Teil des Auswertebereichs auf einen Auswertebildbereich abgebildet wird, wobei der Detektor derart an das Testelement angepasst ist, dass innerhalb des Auswertebildbereichs für jede Dimension eine vorgegebene Mindestzahl von Pixeln vorgesehen ist, wobei die Pixel in einer zweidimensionalen Matrixanordnung angeordnet sind, wobei die Matrixanordnung Pixelzeilen und Pixelspalten aufweist, wobei die Pixelzeilen im Wesentlichen parallel zu einer Längsrichtung des Auswertebereichs und/oder des Auswertebildbereichs angeordnet sind.
Ausführungsform 2: Vorrichtung nach der vorhergehenden Ausführungsform, wobei der Auswertebereich ein Teil des Testelements ist, wobei die Vorrichtung derart eingerichtet ist, dass zum Nachweis des Analyten Körperflüssigkeit auf das Testelement übertragen wird.
Ausführungsform 3: Vorrichtung nach einer der vorhergehenden Ausführungsformen, wobei die Vorrichtung mindestens ein Lanzettenelement mit mindestens einer Kapillare umfasst.
Ausführungsform 4: Vorrichtung nach der vorhergehenden Ausführungsform, wobei die Vorrichtung eingerichtet ist, um mittels der Kapillare Körperflüssigkeit aufzunehmen, wobei die Vorrichtung weiterhin eingerichtet ist, um durch Annäherung der Kapillare an das Testelement Körperflüssigkeit auf das Testelement zu übertragen.
Ausführungsform 5: Vorrichtung nach der vorhergehenden Ausführungsform, wobei der Auswertebereich ein Bereich des Testelements ist, in welchem durch den Übertrag der Körperflüssigkeit auf das Testelement eine optisch nachweisbare Veränderung auftritt.
Ausführungsform 6: Vorrichtung nach einer der drei vorhergehenden Ausführungsformen, wobei die Kapillare eine oder mehrere der folgenden Abmessungen aufweist:
   - eine Breite von 50-200 µm, insbesondere von 90-150 µm und besonders bevorzugt von 120 µm;
   - eine Länge von mindestens 1 mm, insbesondere mindestens 2 mm und vorzugsweise eine Länge von 2-4 mm.
Ausführungsform 7: Vorrichtung nach einer der vorhergehenden Ausführungsformen, wobei die Vorrichtung eingerichtet ist, um den Auswertebereich automatisch zu erkennen.
Ausführungsform 8: Vorrichtung nach der vorhergehenden Ausführungsform, wobei die Vorrichtung eingerichtet ist, um den Auswertebereich nach einem Verfahren zu erkennen, ausgewählt aus der Gruppe bestehend aus:
   - einem Mustererkennungsverfahren, wobei die Vorrichtung mindestens ein Lanzettenelement und/oder mindestens eine Kapillare umfasst, wobei bei dem Mustererkennungsverfahren das Lanzettenelement und/oder die Kapillare der Vorrichtung erkannt werden, wobei als Auswertebereich eine Extrapolation des Lanzettenelements und/oder der Kapillaren auf das Testelement identifiziert wird; und
   - einem Signaländerungsverfahren, wobei als Auswertebereich ein Bereich des Testelements identifiziert wird, innerhalb dessen durch einen Übertrag der Körperflüssigkeit auf das Testelement eine optisch nachweisbare Veränderung eintritt.
Ausführungsform 9: Vorrichtung nach einer der vorhergehenden Ausführungsformen, wobei der Detektor eine Gesamtzahl von maximal 1000 Pixeln aufweist, vorzugsweise eine Gesamtzahl von maximal 500 und besonders bevorzugt eine Gesamtzahl von maximal 256 Pixeln.
Ausführungsform 10: Vorrichtung nach einer der vorhergehenden Ausführungsformen, wobei der Detektor eine Längsseite und eine Breitseite aufweist, insbesondere eine parallel zu einer Kapillaren der Vorrichtung ausgerichtete Längsseite und eine senkrecht zu der Kapillaren ausgerichtete Breitseite, wobei der Detektor in Richtung der Breitseite mindestens 3 Pixelzeilen, vorzugsweise maximal 100 Pixelzeilen, insbesondere 20-50 Pixelzeilen, aufweist, wobei der Detektor weiterhin in Richtung der Längsseite mindestens 1 Pixelspalte, vorzugsweise 2-20 Pixelspalten, insbesondere 5-10 Pixelspalten und besonders bevorzugt 7 Pixelspalten aufweist.
Ausführungsform 11: Vorrichtung nach einer der vorhergehenden Ausführungsformen, wobei in dem Auswertebereichs vorzugsweise mindestens 3 Pixel angeordnet sind, insbesondere 5-30 Pixel und besonders bevorzugt 10 Pixel.
Ausführungsform 12: Vorrichtung nach einer der vorhergehenden Ausführungsformen, wobei der Auswertebereich eine Längsseite und eine Breitseite aufweist, insbesondere eine parallel zu einer Kapillaren der Vorrichtung ausgerichtete Längsseite und eine senkrecht zu der Kapillaren ausgerichtete Breitseite, wobei der Detektor derart ausgestaltet ist, dass innerhalb des Auswertebereichs in Richtung der Breitseite mindestens 3 Pixelzeilen angeordnet sind, insbesondere 3-10 Pixelzeilen, und wobei der Detektor weiterhin derart ausgestaltet ist, dass in Richtung der Längsseite mindestens 1 Pixelspalte, vorzugsweise mindestens 3 Pixelspalten, insbesondere 3-10 Pixelspalten und besonders bevorzugt 7 Pixelspalten angeordnet sind.
Ausführungsform 13: Vorrichtung nach einer der vorhergehenden Ausführungsformen, wobei die Pixel eine lang gestreckte Pixelgeometrie aufweisen, wobei der Auswertebereich eine Längsseite und eine Breitseite aufweist, insbesondere eine parallel zu einer Kapillaren der Vorrichtung ausgerichtete Längsseite und eine senkrecht zu der Kapillaren ausgerichtete Breitseite, wobei die Pixel in Richtung der Längsrichtung eine Länge aufweisen und wobei die Pixel in Richtung der Breitseite eine Breite aufweisen, wobei die Länge die Breite überschreitet, vorzugsweise mindestens um einen Faktor 1,3, insbesondere um mindestens einen Faktor 1,7 oder mindestens einen Faktor 2 und besonders bevorzugt um einen Faktor 2,3.
Ausführungsform 14: Vorrichtung nach einer der vorhergehenden Ausführungsformen, wobei der Detektor eine ortsauflösende Optik aufweist, wobei die ortsauflösende Optik eingerichtet ist, um den Auswertebereich mit einer Vergrößerung von 3:1 bis 0,5:1 auf den Auswertebildbereich abzubilden, vorzugsweise mit einer Vergrößerung von 2:1 bis 0,8:1, besonders bevorzugt mit einer Vergrößerung von 1,1:1 bis 0,9:1 und ideal erweise von 1:1.
Ausführungsform 15: Vorrichtung nach einer der vorhergehenden Ausführungsformen, wobei die Vorrichtung eingerichtet ist, um eine Benetzung des Testelements mit der Körperflüssigkeit zu charakterisieren, insbesondere zu bewerten, wobei die Vorrichtung eingerichtet ist, um die Charakterisierung unter Vergleich mehrerer Pixel in mindestens einer Dimension durchzuführen, vorzugsweise unter Vergleich benachbarter Pixel einer parallel zum Auswertebereich ausgerichteten Pixelzeile.
Ausführungsform 16: Vorrichtung nach einer der vorhergehenden Ausfübrungsformen, wobei die Vorrichtung eingerichtet ist, um einen Leerwert zu erkennen, wobei der Leerwert eine optische Eigenschaft des Bildbereichs und/oder des Auswertebildbereichs ohne Benetzung des Testelements mit Körperflüssigkeit ist, wobei die Vorrichtung eingerichtet ist, um den Leerwert zu bestimmen nach einem Verfahren, ausgewählt aus der Gruppe bestehend aus den folgenden Verfahren:
   - einer Aufnahme einer zeitlichen Bildsequenz, wobei der Auswertebereich bestimmt wird, wobei mindestens ein innerhalb des Auswertebereichs angeordnetes Pixel erkannt und aus der zeitlichen Bildsequenz ein Anfangswert des Pixels bestimmt und als Leerwert verwendet wird;
   - ein Anfangswert der Pixel des Bildbereichs wird gespeichert, aus einer zeitlichen Bildsequenz der Pixel wird der Auswertebereich ermittelt, Pixel außerhalb des Auswertebereichs werden verworfen, und mindestens ein Anfangswert eines Pixels innerhalb des Auswertebereichs wird als Leerwert verwendet;
   - der Auswertebereich wird ermittelt, mindestens ein Pixel außerhalb des Auswertebereichs wird als Leerwert eingesetzt.
Ausführungsform 17: Verfahren zur Erkennung eines Auswertebereichs eines Testelements zum Nachweis mindestens eines Analyten in einer Körperflüssigkeit, insbesondere unter Verwendung einer Vorrichtung nach einer der vorhergehenden Ausführungsformen, wobei mindestens ein Lanzettenelement mit mindestens einer Kapillaren verwendet wird, wobei in der Kapillare aufgenommene Körperflüssigkeit auf das Testelement übertragen wird, wobei mittels mindestens eines ortsaufgelösten optischen Detektors zumindest ein Teil des Testelements auf einen Bildbereich abgebildet wird, wobei zumindest ein Teil des Auswertebereichs des Testelements auf einen Auswertebildbereich abgebildet wird, wobei der Auswertebereich automatisch erkannt wird nach einem Verfahren, ausgewählt aus der Gruppe bestehend aus:
   - einem Mustererkennungsverfahren, wobei bei dem Mustererkennungsverfahren das Lanzettenelement (114) und/oder die Kapillare (116) erkannt wird, wobei als Auswertebereich (136) eine Extrapolation des Lanzettenelements (114) und/oder der Kapillaren (116) auf das Testelement (120) identifiziert wird; und
   - einem Signaländerungsverfahren, wobei als Auswertebereich (136) ein Bereich des Testelements (120) identifiziert wird, innerhalb dessen durch den Übertrag der Körperflüssigkeit auf das Testelement (120) eine optisch nachweisbare Veränderung eintritt.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: ein Ausführungsbeispiel einer erfindungsgemäßen Vorrichtung;
- Figur 2A und 3A: eine Gegenüberstellung einer herkömmlichen Abbildung eines Auswertebereichs (Figur 2A) und einer erfmdungsgemäßen Abbildung (Figur 3A);
- Figuren 2B und 3B: einen Vergleich einer herkömmlichen Vorrichtung (Figur 2B) mit einer erfindungsgemäßen Vorrichtung (Figur 3B) in einer perspektivischen Darstellung;
- Figuren 4 bis 7: Messfehler in verschiedenen Dimensionen bei Verwendung herkömmlicher Detektoren (Figuren 4 und 6) im Vergleich zu erfindungsgemäßen Detektoren (Figuren 5 und 7); und
- Figuren 8A bis 8C: eine Gegenüberstellung eines ordnungsgemäßen Probenübertrags (Figur 8A) und verschiedener Übertragungsfehler (Figuren 8B und 8C).

### Ausführungsbeispiele

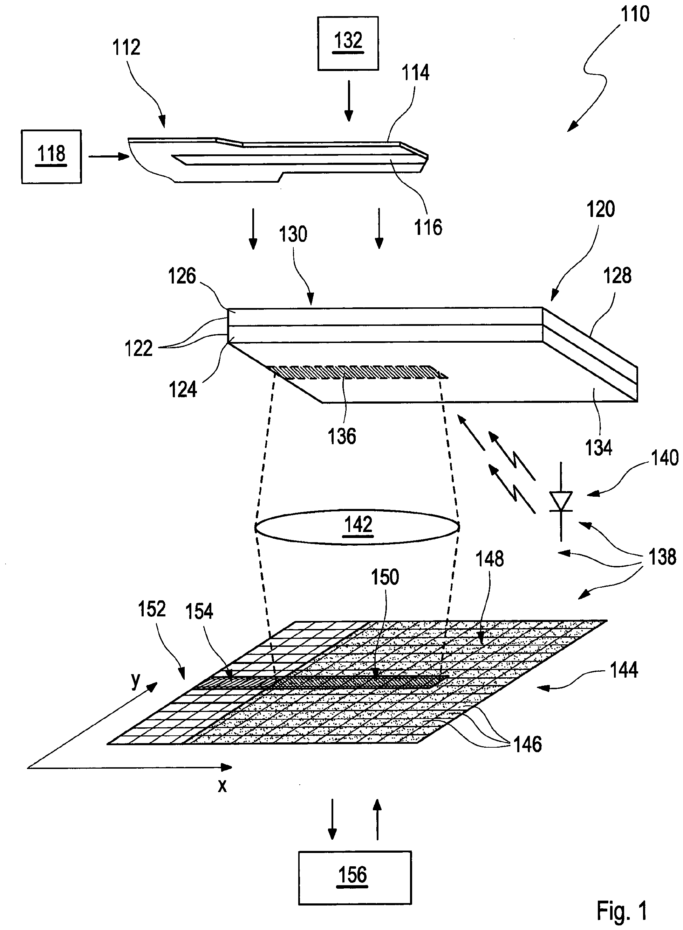

In Figur 1 ist in stark schematisierter Explosionsdarstellung eine erfindungsgemäße Vorrichtung 110 zum Nachweis mindestens eines Analyten in einer Körperflüssigkeit dargestellt. Die Vorrichtung 110 umfasst in dem dargestellten Ausführungsbeispiel einen Microsampler 112 mit einem Lanzettenelement 114 und einer Kapillare 116. Beispielsweise kann es sich um eine metallische Lanzette handeln, in welche die Kapillare 116 als Kapillarspalt eingelassen ist. Das Lanzettenelement 114 kann beispielsweise durch eine Antriebsvorrichtung 118, beispielsweise einen oder mehrere Aktoren (beispielsweise federgetriebene Aktoren) zu einer Stechbewegung angetrieben werden, wobei bei einer Vorwärtsbewegung beispielsweise ein Einstich in eine Haut eines Benutzers erfolgt, und bei einer Rückwärtsbewegung eine Sammlung von Körperflüssigkeit in der Kapillare 116.

Weiterhin umfasst die Vorrichtung 110 in dem dargestellten Ausführungsbeispiel mindestens ein Testelement 120. Dieses Testelement 120 kann insbesondere mindestens ein Testfeld 122 umfassen, beispielsweise ein Testfeld 122 eines Teststreifens und/oder eines Testbands mit mehreren Testfeldern 122 und/oder einer Testscheibe mit mehreren Testfeldern 122. Auch andere Ausgestaltungen sind grundsätzlich möglich. Beispielsweise können mehrere Microsampler vorgesehen sein, denen jeweils mindestens ein Testfeld 122 zugeordnet ist. Beispielsweise können jeweils ein Microsampler 112 und mindestens ein Testfeld 122 in einer Kammer aufgenommen sein und gemeinsam einen Test bilden. Auch andere Ausgestaltungen sind möglich.

Das Testfeld 122 kann beispielsweise eine Nachweisschicht 124 mit mindestens einer Testchemie umfassen, welche bei Anwesenheit des mindestens einen nachzuweisenden Analyten eine optisch nachweisbare und vorzugsweise Analyt-spezifische Reaktion durchführt und/oder nachweisbare Veränderung erfährt. Bezüglich üblicher Testchemien kann beispielsweise auf den oben beschriebenen Stand der Technik verwiesen werden. Weiterhin kann das Testfeld 122 zusätzliche Schichten umfassen, beispielsweise eine oder mehrere Abtrennschichten 126, welche unerwünschte Bestandteile der Probe der Körperflüssigkeit abtrennen, beispielsweise für einen optischen Nachweis störende rote Blutkörperchen, bevor die Probe in die Nachweisschicht 124 gelangt. Weiterhin kann die Abtrennschicht 126 Reflexionseigenschaften aufweisen, beispielsweise indem diese ein oder mehrere reflektierende Stoffe umfasst, beispielsweise weiße Pigmente.

Das Testelement 120 weist eine Probenaufgabeseite 128 auf, auf welcher innerhalb eines Probenaufgabebereichs 130 zumindest ein Teil der in der Kapillaren 116 aufgenommenen Probe der Körperflüssigkeit auf das Testfeld 122 übertragen wird. Zu diesem Zweck kann die Vorrichtung 110 eine Annäherungsvorrichtung 132 umfassen, welche eingerichtet ist, um nach der Probenaufnahme in die Kapillare 116 die Kapillare 116 an den Probenaufgabebereich 130 des Testfelds 122 anzunähern. Die Annäherungsvorrichtung 132 kann beispielsweise einen oder mehrere Aktoren aufweisen, welche aktiv das Lanzettenelement 114 an das Testfeld 122 annähern, beispielsweise auf dieses aufpressen. Alternativ oder zusätzlich kann die Annäherungsvorrichtung 132 jedoch auch mit der Antriebsvorrichtung 118 zusammenwirken, beispielsweise indem durch eine entsprechende Führung des Lanzettenelements 114 beim Zurückziehen des Lanzettenelements 114 nach einer Probenaufnahmebewegung die Kapillare 116 an das Testfeld 122 angenähert wird. Besonders bevorzugt ist es jedoch, wenn die Annäherungsvorrichtung 132 mindestens einen Aktor aufweist, beispielsweise einen Stößel, welcher das Lanzettenelement 114 auf das Testfeld 122 presst, so dass ein definierter Probenaufgabebereich 130 entsteht, welcher auf definierte Weise mit Probe benetzt wird.

Auf der der Probenaufgabeseite 128 gegenüberliegenden Seite des Testelements 120 ist eine Detektionsseite 134 vorgesehen. Nach dem Übertrag der Probe von der Kapillaren 116 auf den Probenaufgabebereich 130 der Probenaufgabeseite 128 bildet sich auf dieser Detektionsseite 134 ein Auswertebereich 136. Beispielsweise kann dieser Auswertebereich 136 eine Projektion des Probenaufgabebereichs 130 bei einer ordnungsgemäßen Übertragung von Körperflüssigkeit aus der Kapillaren 116 auf die Probenaufgabeseite 128 sein. Der Auswertebereich 136 kann somit insbesondere den Bereich der Detektionsseite 134 charakterisieren, innerhalb dessen nach einem ordnungsgemäßen Übertrag von Probe aus der Kapillare 116 auf das Testfeld 122 eine optisch nachweisbare Veränderung auftritt.

Weiterhin umfasst die Vorrichtung 110 in dem dargestellten Ausführungsbeispiel mindestens einen Detektor 138, welcher in dem dargestellten Ausführungsbeispiel aus mehreren Teilen zusammengesetzt ist, die jedoch auch zu einem gemeinsamen Teil, beispielsweise einer Detektorbaugruppe, zusammengefasst sein können. Der Detektor 138 umfasst beispielsweise mindestens eine Lichtquelle 140 zur Beleuchtung der Detektionsseite 134, welche beispielsweise eine Leuchtdiode umfassen kann. Weiterhin umfasst der Detektor 138 eine Optik 142, welche in Figur 1 stark vereinfacht dargestellt ist und welche beispielsweise eine oder mehrere Linsen aufweisen kann. Weiterhin umfasst der Detektor 138 in dem dargestellten Ausführungsbeispiel einen optischen Sensor 144, beispielsweise einen CCD-Chip und/oder CMOS-Chip, welcher eine Mehrzahl von Pixeln 146 in einer Matrixanordnung umfasst. Die Pixel 146 sind vorzugsweise rechteckig ausgestaltet und entlang einer x-Richtung parallel zu einer Längserstreckung der Kapillaren 116 bzw. des Auswertebereichs 136 mit ihrer Längsseite ausgerichtet, und mit ihrer schmaleren Breitseite in einer y-Richtung senkrecht zu dieser Längserstreckungsrichtung. Die Optik 142 ist ausgestaltet, um einen Teil des Testelements 120, insbesondere einen Teil der Detektionsseite 134 des Testelements 120, auf den optischen Sensor 144 abzubilden. Daneben können weitere Teile der Vorrichtung 110 abgebildet werden. So kann beispielsweise, am Rand des Testfelds 122 vorbei, ein Teil des Microsamplers 112, vorzugsweise mitsamt eines Teils der Kapillare 116, durch den Detektor 138 bzw. durch die Optik 142 auf den optischen Sensor 144 abgebildet werden, so dass vorzugsweise ein Teil der Kapillare 116 direkt betrachtet werden kann. Auf diese Weise entstehen vorzugsweise auf dem optischen Sensor 144 mehrere Bereiche. So bildet sich ein in Figur 1 gepunktet dargestellter Bildbereich 148, auf welchen das Testelement 120 und/oder ein Teil dieses Testelements 120, beispielsweise ein Teil der Detektionsseite 134 des Testfelds 122, abgebildet wird. Innerhalb dieses Bildbereichs 148 wird der Auswertebereich 136 auf einen Auswertebildbereich 150 abgebildet, welcher in Figur 1 schraffiert dargestellt ist. Weiterhin bildet sich auf dem optischen Sensor 144 optional ein Bereich, in welchem keine Bestandteile des Testelements 120 abgebildet werden. In diesem Bereich kann beispielsweise ein Abbild 152 des Lanzettenelements 114 entstehen, mit einem Abbild 154 der Kapillaren 116, welches am Rand des Testfelds 120 vorbei aufgenommen wurde. Der Auswertebildbereich 150 stellt somit im Wesentlichen eine Verlängerung dieses Abbilds 154 der Kapillaren 116 dar, wie in Figur 1 symbolisch dargestellt ist.

Weiterhin kann die Vorrichtung 110, insbesondere der Detektor 138, mindestens eine Auswertevorrichtung 156 umfassen, welche in Figur 1 symbolisch angedeutet ist. Diese kann auch ganz oder teilweise in den Detektor 138, beispielsweise in eine Detektorbaugruppe, integriert sein. Die Auswertevorrichtung 156 kann beispielsweise, wie oben dargestellt, mindestens eine Datenverarbeitungsvorrichtung, beispielsweise mindestens einen Microcontroller, und/oder andere elektronische Bauelemente wie beispielsweise Logikbauelemente und/oder Speicherbauelemente umfassen. Die Auswertevorrichtung kann beispielsweise eingerichtet sein, um, gemeinsam mit anderen Bestandteilen der Vorrichtung 110, ein erfindungsgemäßes Verfahren durchzuführen. Die Auswertevorrichtung 156 kann beispielsweise eine Bildauswertung vornehmen.

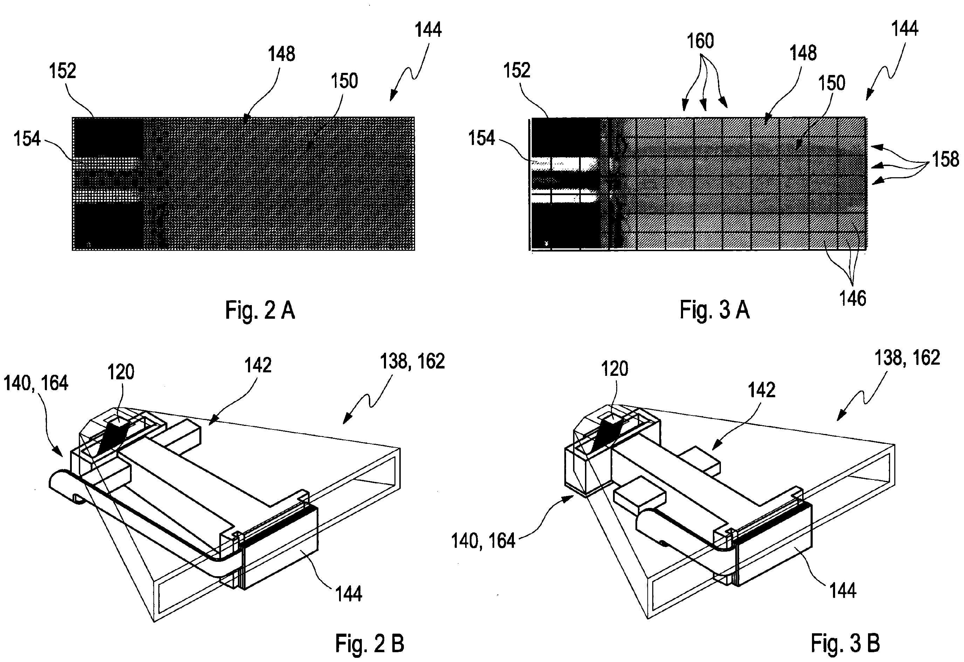

Wie oben dargestellt, besteht ein wesentlicher Gedanke der vorliegenden Erfindung darin, als Detektor 138 einen Detektor mit Makropixeln 146 zu verwenden, also großen Pixeln, im Vergleich zu üblichen CMOS-Kamerasensoren. Dies ist in den Figuren 2A und 2B dargestellt, welche exemplarisch Bilder auf derartigen optischen Sensoren 144 zeigen. Dabei zeigt Figur 2A einen herkömmlichen CMOS-Chip, wohingegen Figur 3A einen optischen Sensor 144 mit "Makropixeln" 146 zeigt, welche im Rahmen der vorliegenden Erfindung besonders bevorzugt sind. Während bei dem CMOS-Sensor 144 gemäß Figur 2A herkömmlicherweise eine Histogrammauswertung erfolgt, beispielsweise wie in EP 1 843 148 A1 beschrieben, kann bei dem Detektor 138 mit den Makropixeln 146 gemäß der erfindungsgemäßen Vorrichtung 110 eine nahezu klassische Auswertung erfolgen, bei welcher beispielsweise die Signale jedes einzelnen Pixels 146 gespeichert und/oder analysiert werden, beispielsweise mit Hilfe der Auswertevorrichtung 156.

**Tabelle 1: Vergleich der Eigenschaften einer herkömmlichen Kameradetektion mit Histogrammanalyse und 3:1-Abbildung (mittlere Spalte) mit einer Makropixeldetektion mit 1:1-Abbildung (rechte Spalte).**

| | **Kamera** | **Makropixel** |
|---|---|---|
| **Abbildung** | | |
| Vergrößerung | 3:1 | 1:1 |
| Toleranzen | + | + |
| Herstellbarkeit | + | + |
| Volumen für Optik und Optoelektronik | 2,57 cm³ | 1,77 cm³ |

| **Optischer Sensor** | | |
|---|---|---|
| Anzahl an Pixeln | ∼65.000 | ≤256 |
| Anzahl an Pixeln mit Glucoseinformation | ∼2500 | ∼10 |
| Pixelgröße | 20 x 20µm² | 30 x 70 µm² |

| **Datentransfer und Datenanalyse** | | |
|---|---|---|
| Vor-Analyse auf Sensorchip | erforderlich | wenn gewünscht |
| Speicherbedarf pro Zyklus (d.h. pro 10 ms ... 100 ms) | 256 x 2 Byte | 256 x 2 Byte |
| Analyseverfahren | Histogrammanalyse | spezieller (einfacher) Algorithmus |

In Tabelle 1 sind herkömmliche Verfahren (Spalte "Kamera") Analyseverfahren unter Verwendung einer erfindungsgemäßen Vorrichtung 110 mit Makropixeln gegenübergestellt. Dabei wurden bei dem herkömmlichen Verfahren Optiken mit einer Vergrößerung von 3:1 verwendet, wie sie typischerweise für Abbildungen mit CMOS-Chips erforderlich sind. Die Anzahl an Pixeln beträgt ca. 65000, wovon ca. 2500 Pixel tatsächlich eine Information über den Analyten (hier als Glucoseinformation bezeichnet) tragen, also Pixel innerhalb des Auswertebildbereichs 150 sind. Die Pixelgröße beträgt typischerweise 20 x 20 µm², und es handelt sich um quadratische Pixel. Bei derartigen Verfahren ist typischerweise eine Voranalyse der Daten auf dem Sensorchip selbst erforderlich, da ansonsten die hohen Bildaufnahmeraten nicht gewährleistet werden können. Bei einer Bildaufnahme alle 10 ms bis 100 ms ergeben sich üblicherweise pro Zyklus Datenmengen von 256 x 2 Byte. Als Analyseverfahren kann beispielsweise eine Histogrammanalyse verwendet werden.

Bei der erfindungsgemäßen Vorrichtung 110 hingegen, welche Makropixel 146 verwendet, erfolgte bei der gezeigten Versuchsreihe eine Abbildung mittels der Optik 142 in einem Vergrößerungsmaßstab 1:1. Während das Volumen für die Optik und die Optoelektronik, also die gesamte Detektorbaugruppe, bei den herkömmlichen Vorrichtungen bei ca. 2,57 cm³ lag, ließ sich erfindungsgemäß bei der Vorrichtung 110 das Volumen für die Optik und die Optoelektronik auf 1,77 cm³ reduzieren. Die Pixelanzahl betrug maximal 256 Pixel. Davon trugen ca. 10 Pixel Glucoseinformation. Die Makropixel 146 wiesen in dem dargestellten Ausführungsbeispiel eine Pixelgröße von 30 x 70 µm² und eine rechteckige Gestalt auf. Eine Vor-Analyse, beispielsweise eine Vorverarbeitung, von Daten auf dem Sensorchip selbst war nicht erforderlich, kann jedoch grundsätzlich durchgeführt werden, wenn dies gewünscht ist. Der Speicherbedarf pro Zyklus ändert sich auch ohne Vorverarbeitung der Daten grundsätzlich nicht. Bei derartig geringen Datenmengen aufgrund der geringen Anzahl an Makropixeln lässt sich ein spezieller, vereinfachter Algorithmus verwenden, um die Konzentration des Analyten in der Körperflüssigkeit zu bestimmen. Beispielsweise kann dieser Algorithmus eine Auswertung sämtlicher Pixel 146, die innerhalb des Auswertebildbereichs 150 angeordnet sind, oder lediglich zentraler Pixel, beinhalten.

Zu diesem Zweck kann beispielsweise zunächst der Auswertebildbereich 150 innerhalb des Bildbereichs 148 erkannt werden, beispielsweise mittels eines der oben beschriebenen Verfahren. So kann beispielsweise eine Verfärbung und/oder eine Grauwertänderung der Makropixel 146 erkannt werden, wodurch der Auswertebildbereich 150 definiert wird. Anschließend können ein oder mehrere vorzugsweise zentral innerhalb des Auswertebildbereichs 150 gelegene Makropixel 146 verwendet werden, um hieraus die Bildinformation auszulesen. Die Erkennung des Auswertebildbereichs 150 kann beispielsweise aufgrund einer Veränderung von Grauwerten erfolgen und/oder aufgrund einer Erkennung des Abbilds 154 der Kapillaren, deren Fortsetzung und/oder Extrapolation in den Bildbereich 148 hinein den Auswertebildbereich 150 darstellt. Beispielsweise können die Pixel 146 in Pixelzeilen 158 parallel zur x-Richtung und somit parallel zur Kapillaren 116 und in Pixelspalten 160 in y-Richtung angeordnet sein. Die am weitesten in der Mitte des Auswertebildbereichs 150 gelegene Pixelzeile 158 kann beispielsweise für die Auswertung herangezogen werden. Alternativ können auch mehrere Pixelzeilen 158 und/oder Teile dieser Pixelzeilen herangezogen werden.

In den Figuren 2B und 3B sind Detektorbaugruppen 162 herkömmlicher Vorrichtungen (Figur 2B) bzw. erfindungsgemäßer Vorrichtungen (Figur 3B) einander gegenübergestellt. Dabei bezeichnet wiederum die Bezugsziffer 120 ein Testelement, beispielsweise ein Testfeld. Das Testelement 120 kann beispielsweise beweglich relativ zur Detektorbaugruppe 162 angeordnet sein, beispielsweise als Teil eines Analysebands. Unterhalb des Testelements 120 ist in dem dargestellten Ausführungsbeispiel eine in den Figuren nicht näher aufgelöste Lichtquelle 140 sowie optional eine Umlenkvorrichtung 164 angeordnet, welche ein reflektiertes Licht hin zu optischen Sensoren 144 lenkt, im Fall der Figur 2B hin zu einem CMOS-Chip mit typischerweise mehr als 10000 Pixeln, und im Fall der erfindungsgemäßen Figur 3B hin zu einem optischen Sensor 144 mit Makropixeln 146, vorzugsweise mit maximal 256 Makropixeln. Im Strahlengang ist weiterhin eine Optik 142 vorgesehen, und zwar bei dem Ausführungsbeispiel gemäß Figur 2B eine Optik mit einer Vergrößerung von 3:1 und dementsprechend einem größeren Bauraum, und im Fall der erfindungsgemäßen Figur 3B eine Optik mit vorzugsweise einer Vergrößerung von 1:1. Wie aus den Figuren 2B und 3B deutlich hervorgeht, ist der Bauraumbedarf der erfindungsgemäßen Ausgestaltung in Figur 3B deutlich geringer als der Bauraumbedarf gemäß Figur 2B.

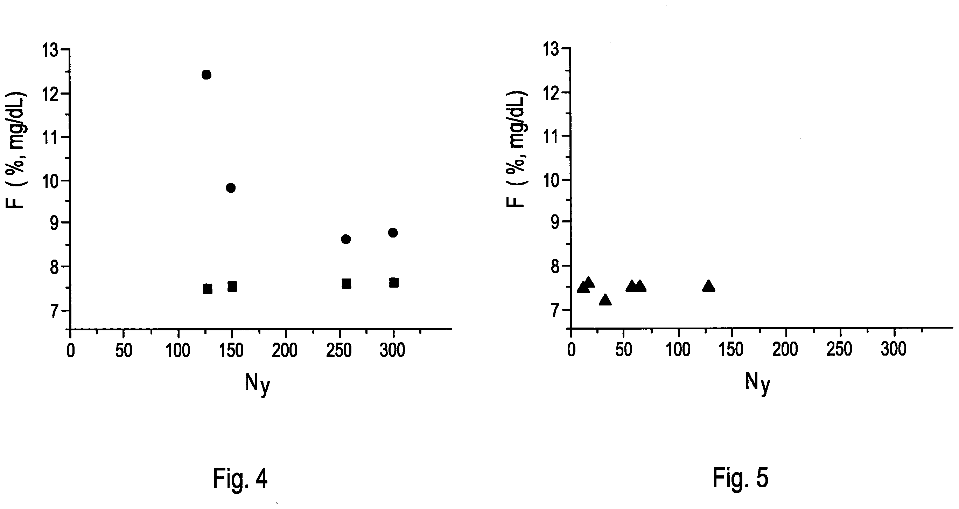

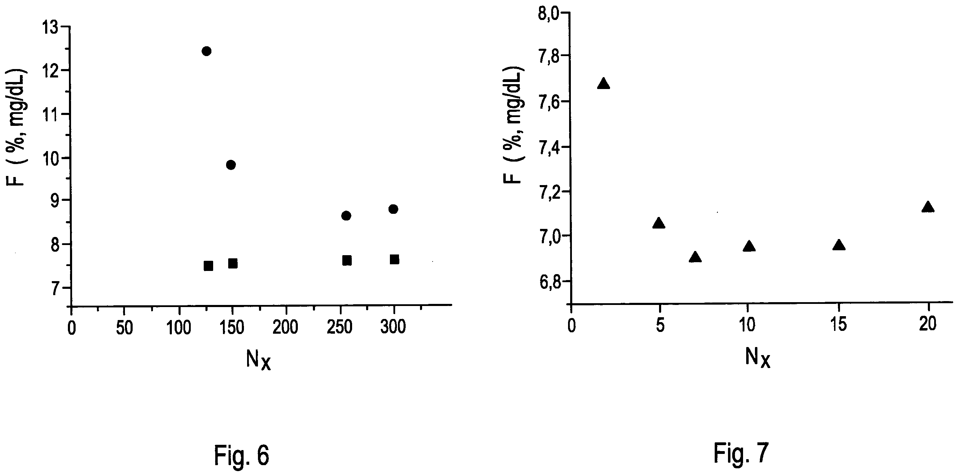

In den Figuren 4 bis 7 sind Vergleichsversuche zwischen herkömmlichen CMOS-Chips gemäß Figur 2A und optischen Sensoren 144 mit Makropixeln 146, beispielsweise gemäß Figur 3A, einander gegenübergestellt. Aufgetragen ist jeweils ein Gesamtfehler einer Glucosekonzentrationsbestimmung in Prozent auf der vertikalen Achse, welcher jeweils mit F bezeichnet ist. Auf der horizontalen Achse ist eine Pixelanzahl des optischen Sensors 144 aufgetragen. Dabei bezeichnet Ny die Anzahl an Pixeln senkrecht zur Kapillaren 116 bzw. deren Abbild (Figuren 4 und 5), also die Anzahl der Pixelzeilen 158 auf dem optischen Sensor 144, und Nₓ die Anzahl der Pixel 146 parallel zur Kapillare 116 bzw. deren Abbild (Figuren 6 und 7), also die Zahl der Pixelspalten 160 pro optischem Sensor 144. Die Figuren 4 und 6 zeigen dabei Experimente mit herkömmlichen CMOS-Sensorchips, wobei die ausgefüllten Kreise Messpunkte darstellen, bei welchen das gesamte Kamerabild ausgewertet wurde. Die ausgefüllten Quadrate bezeichnen Messpunkte, bei welchen zuvor, also vor einer Datenanalyse, zunächst ein auszuwertender Bereich (region of interest, ROI) ausgewählt wurde, innerhalb dessen dann die Auswertung erfolgte. Letzteres erfordert einen erheblichen Bedarf an Zeit, Rechenleistung und damit Ressourcen in der Auswertevorrichtung 156. In den Figuren 5 und 7 sind hingegen Messpunkte für erfindungsgemäße Vorrichtungen 110 mit Makropixeln 146 gezeigt (ausgefüllte Dreiecke). Die Versuche wurden an Kapillaren 116 mit einer Breite von 120 µm durchgeführt. Während bei den herkömmlichen CMOS-Chips eine Pixelgröße von 20 x 20 µm² verwendet wurde, wurden als erfindungsgemäße Makropixel 146 Pixel mit einer Abmessung von 30 x 70 µm² (d.h. 30 µm in y-Richtung und 70 µm in x-Richtung) verwendet, welche, wie in Figur 3A angedeutet, mit ihrer längeren Seite parallel zur Kapillaren 116 ausgerichtet waren.

Wie sich aus dem Vergleich der im Wesentlichen identischen Figuren 6 und 7 ergibt, weisen herkömmliche Sensorchips mit herkömmlichen Auswertungsverfahren erst ab ca. 200 bis 250 Pixeln in x- und in y-Richtung zuverlässige Ergebnisse auf. Bei erfindungsgemäßen Vorrichtungen 110 hingegen mit Makropixeln 146 ergibt sich (man beachte die unterschiedliche Skala der vertikalen Achsen in den Figuren 7 und 6) bereits ab ca. 5 Pixelspalten ein charakteristisches Minimum, und auch bei weniger als 5 Pixelspalten sind bereits geringe Fehler in Figur 7 zu verzeichnen, die mit Fehlern, die in Figur 6 erst ab ca. 250 Pixeln auftreten, vergleichbar sind. Dementsprechend können beispielsweise auch 3 Pixelspalten 160 noch mit hoher Genauigkeit eingesetzt werden. In y-Richtung ist ebenfalls bereits bei sehr kleinen Pixelzahlen bzw. Anzahlen an Pixelzeilen 158 ein sehr geringer Fehler zu verzeichnen, der ebenfalls mit den in Figur 4 erst ab 200 oder 250 Pixeln auftretenden Fehlern vergleichbar ist. So lassen sich beispielsweise, wie aus Figur 5 hervorgeht, optische Sensoren 144 mit 30 Makropixeln 146 in y-Richtung bzw. 30 Pixelzeilen 158 hervorragend einsetzen. Eine detaillierte Analyse hat insbesondere gezeigt, dass bereits optische Sensoren 144 mit 32 Pixelzeilen 158 und 7 Pixelspalten 160, mit Pixelabmessungen von 30 µm x 120 µm hinreichend sind, um eine gute Auswertung zu ermöglichen.

Hierbei ist insbesondere noch zu verzeichnen, dass jeder Pixel 146 aufgrund der hohen Anforderungen an die photometrische Messgenauigkeit typischerweise eine Verschaltung mit jeweils mindestens drei Transistoren benötigt, beispielsweise bei üblichen CMOS-Techniken. Auf einem üblichen Sensor 144, beispielsweise einem CMOS-Chip, sinkt also das Verhältnis von photosensitiver Fläche zur Gesamtfläche pro Pixel einschließlich der Elektronik, also der so genannte Füllfaktor, mit abnehmender Größe der Pixel 146. Bei üblichen CMOS-Chips, wie beispielsweise den in Figur 2A dargestellten Chips, liegt der Füllfaktor typischerweise lediglich zwischen 10 % und 30 %. Mit den vorgeschlagenen Makropixeln 146 hingegen steigt der Füllfaktor schätzungsweise wiederum auf über 80 %, so dass die Signalausbeute höher ist und damit die Zuverlässigkeit, insbesondere das Signal-zu-Rausch-Verhältnis, und/oder ein Strombedarf aufgrund der Möglichkeit, eine Lichtleistung der Lichtquelle 140 bei gleicher Signalqualität herabzusetzen, günstiger sind.

Wie oben beschrieben, kann eine automatische Erkennung des Auswertebereichs 136 erfolgen. Dabei kann der Auswertebereich 136 sowohl in x- als auch in y-Richtung bestimmt werden oder lediglich in einer dieser Richtungen. Besonders günstig ist es, wenn der Auswertebereich 136 innerhalb des Bildbereichs 148 zumindest in y-Richtung, also senkrecht zur Kapillaren 116 bzw. deren Abbild, bestimmt wird. Die vertikale Position der Kapillaren 116 bzw. des Auswertebildbereichs 150 kann dabei insbesondere mit einem einfachen Algorithmus erkannt werden. Dieser kann insbesondere auf einer zeitlichen Differenzbildung für jedes Pixel 146 basieren. Sobald beispielsweise zwei oder mehr in der Figur 3A horizontal benachbarte Pixel 144 die gleiche Änderung erfahren, also eine Änderung, welche (mit Ausnahme eines vorgegebenen Toleranzbereichs von beispielsweise 5 % oder weniger) gleich ist, so kann dann geschlossen werden, dass diese Pixel 146 innerhalb des Auswertebildbereichs 150 angeordnet sind. Bei 32 Pixelzeilen 158 und einem Bildausschnitt von beispielsweise 1 mm und einer Abbildung von 1:1 (was die bevorzugte Lösung ist) entspricht die oben beschriebene Kapillarbreite von 120 µm beispielsweise genau 4 Pixelhöhen, so dass immer mindestens eine oder sogar eigentlich mindestens zwei Pixelzeilen 158 innerhalb des Auswertebildbereichs 150, also des Abbilds der Kapillaren 116, liegen und so von Randeffekten der Kapillare 116, welche beispielsweise auf das Testfeld 122 aufgedrückt werden kann, unabhängige Verfärbung messen können.

Erfindungsgemäß kann weiterhin optional eine frühzeitige Erkennung der Kapillaren 116 und/oder des Auswertebildbereichs 150 durch eine beginnende nachweisbare Veränderung auf der Detektionsseite 134 erfolgen, beispielsweise durch eine beginnende Verfärbung und/oder Abschattung auf der Detektionsseite 134. Dementsprechend kann bereits bevor eine Nachweisreaktion vollständig abgelaufen ist, aus beginnenden Verfärbungen auf den Auswertebildbereich 150 geschlossen werden. Genauere Analysen haben jedoch gezeigt, dass die Kapillare 116 sehr früh, d.h. auch optional vor dem eigentlichen Kontakt zwischen der Probe bzw. der Kapillare 116 und dem Testfeld 122, erkannt werden kann, wenn, wie oben beschrieben, die Detektionsgeometrie des Detektors 138 so ausgelegt ist, dass nicht nur das Testfeld 122 bzw. ein Teil desselben mit seiner darüber liegenden Kapillare 116 vermessen wird, sondern zusätzlich ein schmaler Streifen am Rand die eigentliche Kapillare 116 ohne das Testfeld 122 detektiert. Dies wurde oben anhand der Figur 1 beschrieben. In den Figuren 2A und 3A sind derartige Bereiche, in welchen ein Abbild 154 der Kapillaren 116 außerhalb des Testfelds 122 zu erkennen ist, dargestellt. In diesen Bildern kann die Kapillare 116 sehr einfach und zuverlässig bestimmt werden. Wird das Abbild 154 der Kapillaren 116 detektiert, so kann auf diese Weise durch Extrapolation in Figur 3A nach rechts der Bereich, beispielsweise die Pixelzeilen 158, der zu erwartenden Verfärbung und damit der Auswertebildbereich 150 erkannt bzw. bestimmt werden. Der Vorteil hierbei liegt darin, dass ohne Zwischenspeicherung von Daten ein Leerwert innerhalb des Auswertebildbereichs 150 vor der Benetzung gemessen werden kann. Ohne diese einfache Kapillardetektion muss in der Regel ein vollständiges Leerbild, welches allerdings bei den Makropixeln beispielsweise lediglich nur noch 32 x 7 = 224 Pixel 146 bzw. deren Informationen umfasst, zunächst zwischengespeichert werden, so dass später, d.h. beim Erkennen der Kapillarlage, genau die entsprechende Zeile aus dem Leerbild zur Ermittlung des Leerwerts herangezogen werden kann.

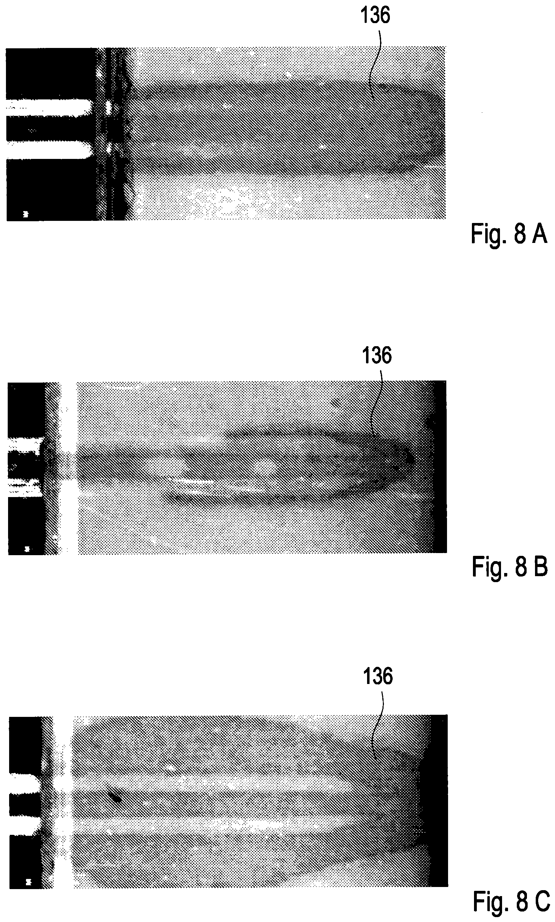

Weiterhin kann die Vorrichtung 110 auch, wie oben beschrieben, eingerichtet sein, um einen Übertrag der Probe von dem Microsampler 112 auf das Testelement 120 zu charakterisieren. Insbesondere kann diese Charakterisierung derart ausgestaltet sein, dass dabei ein korrekter Probenübertrag von Übertragungsfehlern oder Befüllungsfehlern unterschieden wird. Dies ist in den Figuren 8A bis 8C dargestellt. Während Figur 8A eine korrekte Befüllung der Kapillaren 116 zeigt, gefolgt von einem korrekten Übertrag auf den Auswertebereich 136, zeigt Figur 8B einen Fall, in welchem die Kapillare 116 unvollständig gefüllt wurde und/oder bei welchem ein unvollständiger Übertrag von Probe aus der Kapillaren 116 auf den Probenaufgabebereich 130 erfolgte, also eine Unterbenetzung. Figur 8C zeigt hingegen einen Fall, in welchem ein Überlaufen erfolgt, also eine Überbenetzung oder Überflutung.

Eine derartige Erkennung von Fehlern kann im Rahmen der vorgeschlagenen Vorrichtung 110 mit den Makropixeln 146, welche herstellungstechnisch leicht realisierbar sind, beispielsweise mittels einer einfachen Logikabfrage erfolgen. So kann beispielsweise eine Logikabfrage durchgeführt werden, ob alle Pixel 146 innerhalb einer Pixelzeile 158 den gleichen Grauwert bzw. das gleiche Signal aufweisen, beispielsweise innerhalb einer engen Fehlertoleranz von z.B. weniger als 5 %. Auf diese Weise können Unterbenetzungen gemäß Figur 8B erkannt werden. Weiterhin kann, zur Erkennung von Überflutungen gemäß Figur 8C, abgefragt werden, ob beispielsweise 10 Pixelzeilen 158 oberhalb der Kapillaren 116 bzw. deren Abbild auf dem optischen Sensor 144 und/oder um einen anderen vorgegebenen Versatz im Abbild, nach Benetzung ein unterschiedlicher Grauwert bzw. ein unterschiedliches Signal entsteht oder nicht. Bei dem in Figur 8C gezeigten Fall wäre dies beispielsweise nicht der Fall. Wird keine Abweichung erkannt, so kann hieraus dann auf ein Überfluten gemäß Figur 8C geschlossen werden. Auch andere Algorithmen können jedoch grundsätzlich zur Erkennung von Benetzungsfehlern eingesetzt werden.

### Bezugszeichenliste

- 110: Vorrichtung zum Nachweis eines Analyten
- 112: Microsampler
- 114: Lanzettenelement
- 116: Kapillare
- 118: Antriebsvorrichtung
- 120: Testelement
- 122: Testfeld
- 124: Nachweisschicht
- 126: Abtrennschicht
- 128: Probenaufgabeseite
- 130: Probenaufgabebereich
- 132: Annäherungsvorrichtung
- 134: Detektionsseite
- 136: Auswertebereich
- 138: Detektor
- 140: Lichtquelle
- 142: Optik
- 144: optischer Sensor
- 146: Pixel 148 Bildbereich
- 150: Auswertebildbereich
- 152: Abbild des Lanzettenelements
- 154: Abbild der Kapillaren
- 156: Auswertevorrichtung
- 158: Pixelzeilen 160 Pixelspalten
- 162: Detektorbaugruppe
- 164: Umlenkvorrichtung

## Patentansprüche

1. Vorrichtung (110) zum Nachweis mindestens eines Analyten in einer Körperflüssigkeit, umfassend mindestens ein Testelement (120) mit mindestens einem zweidimensionalen Auswertebereich (136), weiterhin umfassend mindestens einen ortsauflösenden optischen Detektor (138) mit einer Mehrzahl von Pixeln (146), wobei der Detektor (138) eingerichtet ist, um zumindest einen Teil des Testelements (120) auf einen Bildbereich (148) abzubilden, wobei zumindest ein Teil des Auswertebereichs (136) auf einen Auswertebildbereich (150) abgebildet wird, wobei der Detektor (138) derart an das Testelement (120) angepasst ist, dass innerhalb des Auswertebildbereichs (150) für jede Dimension eine vorgegebene Mindestzahl von Pixeln (146) vorgesehen ist, wobei die Pixel (146) in einer Matrixanordnung angeordnet sind, wobei die Matrixanordnung Pixelzeilen (158) und mindestens eine Pixelspalte (160) aufweist, wobei die Pixelzeilen (158) im Wesentlichen parallel zu einer Längsrichtung des Auswertebereichs (136) und/oder des Auswertebildbereichs (150) angeordnet sind, wobei die Vorrichtung (110) mindestens ein Lanzettenelement (114) mit mindestens einer Kapillare (116) umfasst wobei in der Kapillare (116) aufgenommene Körperflüssigkeit auf das Testelement (120) übertragen wird, wobei der Detektor (138) eine Längsseite und eine Breitseite aufweist, wobei die Längsseite parallel zu der Kapillaren (116) der Vorrichtung (110) ausgerichtet ist und wobei die Breitseite senkrecht zu der Kapillaren (116) ausgerichtet ist, wobei der Detektor (138) in Richtung der Breitseite mindestens 3 Pixelzeilen (158) aufweist, wobei der Detektor (138) weiterhin in Richtung der Längsseite mindestens 1 Pixelspalte (160) aufweist.

2. Vorrichtung (110) nach dem vorhergehenden Anspruch, wobei der Auswertebereich (136) ein Teil des Testelements (120) ist, wobei die Vorrichtung (110) derart eingerichtet ist, dass zum Nachweis des Analyten Körperflüssigkeit auf das Testelement (120) übertragen wird.

3. Vorrichtung (110) nach einem der vorhergehenden Ansprüche, wobei die Vorrichtung (110) eingerichtet ist, um mittels der Kapillare (116) Körperflüssigkeit aufzunehmen, wobei die Vorrichtung (110) weiterhin eingerichtet ist, um durch Annäherung der Kapillare (116) an das Testelement (120) Körperflüssigkeit auf das Testelement (120) zu übertragen.

4. Vorrichtung (110) nach dem vorhergehenden Anspruch, wobei der Auswertebereich (136) ein Bereich des Testelements (120) ist, in welchem durch den Übertrag der Körperflüssigkeit auf das Testelement (120) eine optisch nachweisbare Veränderung auftritt.

5. Vorrichtung (110) nach einem der vorhergehenden Ansprüche, wobei die Kapillare (116) eine oder mehrere der folgenden Abmessungen aufweist:
- eine Breite von 50-200 µm, insbesondere von 90-150 µm und besonders bevorzugt von 120 µm;
- eine Länge von mindestens 1 mm, insbesondere mindestens 2 mm und vorzugsweise eine Länge von 2-4 mm.

6. Vorrichtung (110) nach einem der vorhergehenden Ansprüche, wobei die Vorrichtung (110) eingerichtet ist, um den Auswertebereich (136) automatisch zu erkennen.

7. Vorrichtung (110) nach dem vorhergehenden Anspruch, wobei die Vorrichtung (110) eingerichtet ist, um den Auswertebereich (136) nach einem Verfahren zu erkennen, ausgewählt aus der Gruppe bestehend aus:
- einem Mustererkennungsverfahren, wobei bei dem Mustererkennungsverfahren das Lanzettenelement (114) und/oder die Kapillare (116) der Vorrichtung (110) erkannt werden, wobei als Auswertebereich (136) eine Extrapolation des Lanzettenelements (116) und/oder der Kapillaren (116) auf das Testelement (120) identifiziert wird; und
- einem Signaländerungsverfahren, wobei als Auswertebereich (136) ein Bereich des Testelements (120) identifiziert wird, innerhalb dessen durch einen Übertrag der Körperflüssigkeit auf das Testelement (120) eine optisch nachweisbare Veränderung eintritt.

8. Vorrichtung (110) nach einem der vorhergehenden Ansprüche, wobei der Detektor (138) eine Gesamtzahl von maximal 1000 Pixeln (146) aufweist, vorzugsweise eine Gesamtzahl von maximal 500 und besonders bevorzugt eine Gesamtzahl von maximal 256 Pixeln (146).

9. Vorrichtung (110) nach einem der vorhergehenden Ansprüche, wobei in dem Auswertebereichs (136) vorzugsweise mindestens 3 Pixel (146) angeordnet sind, insbesondere 5-30 Pixel (146) und besonders bevorzugt 10 Pixel (146).

10. Vorrichtung (110) nach einem der vorhergehenden Ansprüche, wobei der Auswertebereich (136) eine Längsseite und eine Breitseite aufweist, insbesondere eine parallel zu einer Kapillaren (116) der Vorrichtung (110) ausgerichtete Längsseite und eine senkrecht zu der Kapillaren (116) ausgerichtete Breitseite, wobei der Detektor (138) derart ausgestaltet ist, dass innerhalb des Auswertebereichs (136) in Richtung der Breitseite mindestens 3 Pixelzeilen (158) angeordnet sind, insbesondere 3-10 Pixelzeilen (158), und wobei der Detektor (138) weiterhin derart ausgestaltet ist, dass in Richtung der Längsseite mindestens 1 Pixelspalte (160), vorzugsweise mindestens 3 Pixelspalten (160), insbesondere 3-10 Pixelspalten (160) und besonders bevorzugt 7 Pixelspalten (160) angeordnet sind.

11. Vorrichtung (110) nach einem der vorhergehenden Ansprüche, wobei die Pixel (146) eine lang gestreckte Pixelgeometrie aufweisen, wobei der Auswertebereich (136) eine Längsseite und eine Breitseite aufweist, insbesondere eine parallel zu einer Kapillaren (116) der Vorrichtung (110) ausgerichtete Längsseite und eine senkrecht zu der Kapillaren (116) ausgerichtete Breitseite, wobei die Pixel (146) in Richtung der Längsrichtung eine Länge aufweisen und wobei die Pixel (146) in Richtung der Breitseite eine Breite aufweisen, wobei die Länge die Breite überschreitet, vorzugsweise mindestens um einen Faktor 1,3, insbesondere um mindestens einen Faktor 1,7 oder mindestens einen Faktor 2 und besonders bevorzugt um einen Faktor 2,3.

12. Vorrichtung (110) nach einem der vorhergehenden Ansprüche, wobei der Detektor (138) eine ortsauflösende Optik (142) aufweist, wobei die ortsauflösende Optik (142) eingerichtet ist, um den Auswertebereich (136) mit einer Vergrößerung von 3:1 bis 0,5:1 auf den Auswertebildbereich (150) abzubilden, vorzugsweise mit einer Vergrößerung von 2:1 bis 0,8:1, besonders bevorzugt mit einer Vergrößerung von 1,1:1 bis 0,9:1 und idealerweise von 1:1.

13. Vorrichtung (110) nach einem der vorhergehenden Ansprüche, wobei die Vorrichtung (110) eingerichtet ist, um eine Benetzung des Testelements (120) mit der Körperflüssigkeit zu charakterisieren, insbesondere zu bewerten, wobei die Vorrichtung (110) eingerichtet ist, um die Charakterisierung unter Vergleich mehrerer Pixel (146) in mindestens einer Dimension durchzuführen, vorzugsweise unter Vergleich benachbarter Pixel (146) einer parallel zum Auswertebereich (136) ausgerichteten Pixelzeile (158).

14. Vorrichtung (110) nach einem der vorhergehenden Ansprüche, wobei die Vorrichtung (110) eingerichtet ist, um einen Leerwert zu erkennen, wobei der Leerwert eine optische Eigenschaft des Bildbereichs (148) und/oder des Auswertebildbereichs (150) ohne Benetzung des Testelements (120) mit Körperflüssigkeit ist, wobei die Vorrichtung (110) eingerichtet ist, um den Leerwert zu bestimmen nach einem Verfahren, ausgewählt aus der Gruppe bestehend aus den folgenden Verfahren:
- einer Aufnahme einer zeitlichen Bildsequenz, wobei der Auswertebereich (136) bestimmt wird, wobei mindestens ein innerhalb des Auswertebereichs (136) angeordnetes Pixel (146) erkannt und aus der zeitlichen Bildsequenz ein Anfangswert des Pixels (146) bestimmt und als Leerwert verwendet wird;
- ein Anfangswert der Pixel (146) des Bildbereichs (148) wird gespeichert, aus einer zeitlichen Bildsequenz der Pixel (146) wird der Auswertebereich (136) ermittelt, Pixel (146) außerhalb des Auswertebereichs (136) werden verworfen, und mindestens ein Anfangswert eines Pixels (146) innerhalb des Auswertebereichs (136) wird als Leerwert verwendet; und
- der Auswertebereich (136) wird ermittelt, mindestens ein Pixel (146) außerhalb des Auswertebereichs (136) wird als Leerwert eingesetzt.

15. Verfahren zur Erkennung eines zweidimensionalen Auswertebereichs (136) eines Testelements (120) zum Nachweis mindestens eines Analyten in einer Körperflüssigkeit, wobei mindestens ein Lanzettenelement (114) mit mindestens einer Kapillaren (116) verwendet wird, wobei in der Kapillare (116) aufgenommene Körperflüssigkeit auf das Testelement (120) übertragen wird, wobei mittels mindestens eines ortsaufgelösten optischen Detektors (138) mit einer Mehrzahl von in einer Matrixanordnung ongeordneten Pixeln, zumindest ein Teil des Testelements (120) auf einen Bildbereich (148) abgebildet wird, wobei der Detektor (138) eine Längsseite und eine Breitseite aufweist, wobei die Längsseite parallel zu der Kapillaren (116) der Vorrichtung (110) ausgerichtet ist und wobei die Breitseite senkrecht zu der Kapillaren (116) ausgerichtet ist, wobei der Detektor (138) in Richtung der Breitseite mindestens 3 Pixelzeilen (158) aufweist, wobei der Detektor (138) weiterhin in Richtung der Längsseite mindestens 1 Pixelspalte (160) aufweist, wobei zumindest ein Teil des Auswertebereichs (136) des Testelements (120) auf einen Auswertebildbereich (150) abgebildet wird, wobei der Auswertebereich (136) automatisch erkannt wird nach einem Verfahren, ausgewählt aus der Gruppe bestehend aus:
- einem Mustererkennungsverfahren, wobei bei dem Mustererkennungsverfahren das Lanzettenelement (114) und/oder die Kapillare (116) erkannt wird, wobei als Auswertebereich (136) eine Extrapolation des Lanzettenelements (114) und/oder der Kapillaren (116) auf das Testelement (120) identifiziert wird; und
- einem Signaländerungsverfahren, wobei als Auswertebereich (136) ein Bereich des Testelements (120) identifiziert wird, innerhalb dessen durch den Übertrag der Körperflüssigkeit auf das Testelement (120) eine optisch nachweisbare Veränderung eintritt.

## Claims

1. Device (110) for detecting at least one analyte in a bodily fluid, comprising at least one test element (120) with at least one two-dimensional evaluation region (136), furthermore comprising at least one spatially resolving optical detector (138) having a plurality of pixels (146), wherein the detector (138) is designed to image at least part of the test element (120) onto an image region (148), wherein at least part of the evaluation region (136) is imaged onto an evaluation image region (150), wherein the detector (138) is matched to the test element (120) such that a predetermined minimum number of pixels (146) is provided for each dimension within the evaluation image region (150), wherein the pixels (146) are arranged in a matrix arrangement, wherein the matrix arrangement has pixel rows (158) and at least one pixel column (160), wherein the pixel rows (158) are arranged substantially parallel to a longitudinal direction of the evaluation region (136) and/or of the evaluation image region (150), wherein the device (110) comprises at least one lancet element (114) with at least one capillary (116), wherein bodily fluid taken up into the capillary (116) is transferred onto the test element (120), wherein the detector (138) has a longitudinal side and a transverse side, wherein the longitudinal side is aligned parallel to the capillary (116) of the device (110) and wherein the transverse side is arranged perpendicular to the capillary (116), wherein the detector (138) has at least 3 pixel rows (158) in the direction of the transverse side, wherein the detector (138) furthermore has at least 1 pixel column (160) in the direction of the longitudinal side.

2. Device (110) according to the preceding claim, wherein the evaluation region (136) is part of the test element (120), wherein the device (110) is embodied such that bodily fluid is transferred onto the test element (120) for detecting the analyte.

3. Device (110) according to one of the preceding claims, wherein the device (110) is designed to take up bodily fluid by means of the capillary (116), wherein the device (110) is furthermore designed to transfer bodily fluid onto the test element (120) by causing the capillary (116) to approach the test element (120).

4. Device (110) according to the preceding claim, wherein the evaluation region (136) is a region of the test element (120), in which an optically detectable change occurs as a result of transferring the bodily fluid onto the test element (120).

5. Device (110) according to one of the preceding claims, wherein the capillary (116) has one or more of the following dimensions:
- a width of 50-200 µm, more particularly of 90-150 µm and particularly preferably of 120 µm;
- a length of at least 1 mm, more particularly of at least 2 mm and preferably a length of 2-4 mm.

6. Device (110) according to one of the preceding claims, wherein the device (110) is designed to recognize the evaluation region (136) automatically.

7. Device (110) according to the preceding claim, wherein the device (110) is designed to recognize the evaluation region (136) according to a method selected from the group consisting of:
- a pattern recognition method, wherein, in the pattern recognition method, the lancet element (114) and/or the capillary (116) of the device (110) are recognized, wherein an extrapolation of the lancet element (116) and/or of the capillary (116) onto the test element (120) is identified as evaluation region (136); and
- a signal-change method, wherein a region of the test element (120) within which an optically detectable change occurs as a result of a transfer of the bodily fluid onto the test element (120) is identified as evaluation region (136).

8. Device (110) according to one of the preceding claims, wherein the detector (138) has a total number of no more than 1000 pixels (146), preferably a total number of no more than 500 and particularly preferably a total number of no more than 256 pixels (146).

9. Device (110) according to one of the preceding claims, wherein preferably at least 3 pixels (146), more particularly 5-30 pixels (146) and particularly preferably 10 pixels (146) are arranged in the evaluation region (136).

10. Device (110) according to one of the preceding claims, wherein the evaluation region (136) has a longitudinal side and a transverse side, more particularly a longitudinal side aligned parallel to a capillary (116) of the device (110) and a transverse side arranged perpendicular to the capillary (116), wherein the detector (138) is designed such that at least 3 pixel rows (158), more particularly 3-10 pixel rows (158) are arranged in the direction of the transverse side within the evaluation region (136), and wherein the detector (138) is furthermore designed such that at least 1 pixel column (160), preferably at least 3 pixel columns (160), more particularly 3-10 pixel columns (160) and particularly preferably 7 pixel columns (160) are arranged in the direction of the longitudinal side.

11. Device (110) according to one of the preceding claims, wherein the pixels (146) have an elongate pixel geometry, wherein the evaluation region (136) has a longitudinal side and a transverse side, more particularly a longitudinal side aligned parallel to a capillary (116) of the device (110) and a transverse side arranged perpendicular to the capillary (116), wherein the pixels (146) have a length in the direction of the longitudinal direction and wherein the pixels (146) have a width in the direction of the transverse side, wherein the length exceeds the width, preferably by at least a factor of 1.3, more particularly by at least a factor of 1.7 or at least a factor of 2 and particularly preferably by a factor of 2.3.

12. Device (110) according to one of the preceding claims, wherein the detector (138) has a spatially resolving optical unit (142), wherein the spatially resolving optical unit (142) is designed to image the evaluation region (136) onto the evaluation image region (150) with a magnification of 3:1 to 0.5:1, preferably with a magnification of 2:1 to 0.8:1, particularly preferably with a magnification of 1.1:1 to 0.9:1 and ideally of 1:1.

13. Device (110) according to one of the preceding claims, wherein the device (110) is designed to characterize, more particularly evaluate, a wetting of the test element (120) with the bodily fluid, wherein the device (110) is designed to carry out the characterization by comparing a plurality of pixels (146) in at least one dimension, preferably by comparing adjacent pixels (146) of a pixel row (158) aligned parallel to the evaluation region (136).

14. Device (110) according to one of the preceding claims, wherein the device (110) is designed to recognize a blank value, wherein the blank value is an optical property of the image region (148) and/or of the evaluation image region (150) without wetting of the test element (120) with bodily fluid, wherein the device (110) is designed to determine the blank value according to a method, selected from the group consisting of the following methods:
- recording a temporal image sequence, wherein the evaluation region (136) is determined, wherein at least one pixel (146) arranged within the evaluation region (136) is recognized and an initial value of the pixel (146) is determined from the temporal image sequence and used as blank value;
- an initial value of the pixels (146) of the image region (148) is stored, the evaluation region (136) is established from a temporal image sequence of the pixels (146), pixels (146) outside of the evaluation region (136) are discarded and at least one initial value of a pixel (146) within the evaluation region (136) is used as blank value; and
- the evaluation region (136) is established, at least one pixel (146) from outside of the evaluation region (136) is used as blank value.

15. Method for recognizing a two-dimensional evaluation region (136) of a test element (120) for detecting at least one analyte in a bodily fluid, wherein use is made of at least one lancet element (114) with at least one capillary (116), wherein bodily fluid taken up into the capillary (116) is transferred onto the test element (120), wherein at least one spatially resolved optical detector (138) having a plurality of pixels arranged in a matrix arrangement is used to image at least part of the test element (120) onto an image region (148), wherein the detector (138) has a longitudinal side and a transverse side, wherein the longitudinal side is aligned parallel to the capillary (116) of the device (110) and the transverse side is arranged perpendicular to the capillary (116), wherein the detector (138) has at least 3 pixel rows (158) in the direction of the transverse side, wherein the detector (138) furthermore has at least 1 pixel column (160) in the direction of the longitudinal side, wherein at least part of the evaluation region (136) of the test element (120) is imaged onto an evaluation image region (150), wherein the evaluation region (136) is automatically recognized according to a method selected from the group consisting of:
- a pattern recognition method, wherein, in the pattern recognition method, the lancet element (114) and/or the capillary (116) are recognized, wherein an extrapolation of the lancet element (114) and/or of the capillary (116) onto the test element (120) is identified as evaluation region (136); and
- a signal-change method, wherein a region of the test element (120) within which an optically detectable change occurs as a result of the transfer of the bodily fluid onto the test element (120) is identified as evaluation region (136).

## Revendications

1. Dispositif (110) pour déceler au moins un analyte dans un liquide corporel, comprenant au moins un élément de test (120) doté d'au moins une zone d'interprétation bidimensionnelle (136), comprenant en outre au moins un détecteur optique (138) à résolution locale ayant une pluralité de pixels (146), le détecteur (138) étant conçu pour représenter au moins une partie de l'élément de test (120) sur une zone d'image (148), au moins une partie de la zone d'interprétation (136) étant représentée sur une zone d'image d'interprétation (150), le détecteur (138) étant adapté à l'élément de test (120) de telle sorte qu'un nombre minimum de pixels (146) est présent à l'intérieur de la zone d'image d'interprétation (150) pour chaque dimension, les pixels (146) étant disposés en un arrangement matriciel, l'arrangement matriciel possédant des lignes de pixels (158) et au moins une colonne de pixels (160), les lignes de pixels (158) étant disposées sensiblement en parallèle à une direction longitudinale de la zone d'interprétation (136) et/ou de la zone d'image d'interprétation (150), le dispositif (110) comprenant au moins un élément à lancette (114) doté d'au moins un capillaire (116), le liquide corporel absorbé dans le capillaire (116) étant transféré sur l'élément de test (120), le détecteur (138) possédant un côté long et un côté large, le côté long étant orienté parallèlement aux capillaires (116) du dispositif (110) et le côté large étant orienté perpendiculairement aux capillaires (116), le détecteur (138) possédant au moins 3 lignes de pixels (158) dans la direction du côté large, le détecteur (138) possédant en outre au moins une colonne de pixels (160) dans la direction du côté long.

2. Dispositif (110) selon la revendication précédente, la zone d'interprétation (136) étant une partie de l'élément de test (120), le dispositif (110) étant conçu de telle sorte que pour déceler l'analyte, du liquide corporel est transféré sur l'élément de test (120).

3. Dispositif (110) selon l'une des revendications précédentes, le dispositif (110) étant conçu pour absorber du liquide corporel au moyen du capillaire (116), le dispositif (110) étant en outre conçu pour transférer du liquide corporel sur l'élément de test (120) par approche du capillaire (116) de l'élément de test (120).

4. Dispositif (110) selon la revendication précédente, la zone d'interprétation (136) étant une zone de l'élément de test (120) dans laquelle, du fait du transfert du liquide corporel sur l'élément de test (120), il se produit une modification décelable optiquement.

5. Dispositif (110) selon l'une des revendications précédentes, le capillaire (116) possédant une ou plusieurs des dimensions suivantes :
- une largeur de 50 à 200 µm, notamment de 90 à 150 µm et particulièrement de préférence de 120 µm ;
- une longueur d'au moins 1 mm, notamment d'au moins 2 mm et de préférence une longueur de 2 à 4 mm.

6. Dispositif (110) selon l'une des revendications précédentes, le dispositif (110) étant conçu pour reconnaître automatiquement la zone d'interprétation (136).

7. Dispositif (110) selon la revendication précédente, le dispositif (110) étant conçu pour reconnaître la zone d'interprétation (136) conformément à un procédé choisi dans le groupe composé de :
- un procédé de reconnaissance de modèle, ledit procédé de reconnaissance de modèle comprenant la reconnaissance de l'élément à lancette (114) et/ou du capillaire (116) du dispositif (110), la zone d'interprétation (136) identifiée étant une extrapolation de l'élément à lancette (116) et/ou des capillaires (116) sur l'élément de test (120) ; et
- un procédé de modification de signal, la zone d'interprétation (136) identifiée étant une zone de l'élément de test (120) à l'intérieur de laquelle, du fait d'un transfert du liquide corporel sur l'élément de test (120), il se produit une modification décelable optiquement.

8. Dispositif (110) selon l'une des revendications précédentes, le détecteur (138) possédant un nombre total maximal de 1000 pixels (146), de préférence un nombre total maximal de 500 et notamment de préférence un nombre total maximal de 256 pixels (146).

9. Dispositif (110) selon l'une des revendications précédentes, de préférence un minimum de 3 pixels (146) étant disposés dans la zone d'interprétation (136), notamment de 5 à 30 pixels (146) et notamment de préférence 10 pixels (146).

10. Dispositif (110) selon l'une des revendications précédentes, la zone d'interprétation (136) possédant un côté long et un côté large, notamment un côté long orienté parallèlement aux capillaires (116) du dispositif (110) et un côté large orienté perpendiculairement aux capillaires (116), le détecteur (138) étant configuré de telle sorte qu'un minimum de 3 lignes de pixels (158) sont disposées à l'intérieur de la zone d'interprétation (136) dans la direction du côté large, notamment de 3 à 10 lignes de pixels (158), et le détecteur (138) étant en outre configuré de telle sorte qu'un minimum d'une colonne de pixels (160), de préférence au moins 3 colonnes de pixels (160), notamment de 3 à 10 colonnes de pixels (160) et particulièrement de préférence 7 colonnes de pixels (160) sont disposées dans la direction du côté long.

11. Dispositif (110) selon l'une des revendications précédentes, les pixels (146) possédant une géométrie de pixel étendue en longueur, la zone d'interprétation (136) possédant un côté long et un côté large, notamment un côté long orienté parallèlement aux capillaires (116) du dispositif (110) et un côté large orienté perpendiculairement aux capillaires (116), les pixels (146) dans la direction du côté long possédant une longueur et les pixels (146) dans la direction du côté large possédant une largeur, la longueur étant supérieure à la largeur, de préférence d'au moins un facteur 1,3, notamment d'au moins un facteur 1,7 ou d'au moins un facteur 2 et particulièrement de préférence d'un facteur 2,3.

12. Dispositif (110) selon l'une des revendications précédentes, le détecteur (138) possédant une optique (142) à résolution locale, l'optique (142) à résolution locale étant conçue pour représenter la zone d'interprétation (136) avec un grossissement de 3:1 à 0,5:1 sur la zone d'image d'interprétation (150), de préférence avec un grossissement de 2:1 à 0,8:1, particulièrement de préférence avec un grossissement de 1,1:1 à 0,9:1 et idéalement de 1:1.

13. Dispositif (110) selon l'une des revendications précédentes, le dispositif (110) étant conçu pour caractériser, notamment estimer, un mouillage de l'élément de test (120) avec le liquide corporel, le dispositif (110) étant conçu pour effectuer la caractérisation en comparant plusieurs pixels (146) dans au moins une dimension, de préférence en comparant les pixels (146) voisins d'une ligne de pixels (158) orientée parallèlement à la zone d'interprétation (136).

14. Dispositif (110) selon l'une des revendications précédentes, le dispositif (110) étant conçu pour reconnaître une valeur témoin, la valeur témoin étant une propriété optique de la zone d'image (148) et/ou de la zone d'image d'interprétation (150) sans mouillage de l'élément de test (120) avec le liquide corporel, le dispositif (110) étant conçu pour déterminer la valeur témoin conformément à un procédé choisi dans le groupe composé des procédés suivantes :
- un enregistrement d'une séquence d'images chronologique, la zone d'interprétation (136) étant définie, au moins un pixel (146) disposé à l'intérieur de la zone d'interprétation (136) étant reconnu et une valeur initiale du pixel (146) étant définie à partir de la séquence d'images chronologique et utilisée comme valeur témoin ;
- une valeur initiale du pixel (146) de la zone d'image (148) est mise en mémoire, la zone d'interprétation (136) est déterminée à partir d'une séquence d'images chronologique des pixels (146), les pixels (146) en-dehors de la zone d'interprétation (136) sont rejetés et au moins une valeur initiale d'un pixel (146) à l'intérieur de la zone d'interprétation (136) est utilisée comme valeur témoin ; et
- la zone d'interprétation (136) est déterminée, au moins un pixel (146) en-dehors de la zone d'interprétation (136) est utilisé comme valeur témoin.

15. Procédé de reconnaissance d'une zone d'interprétation (136) bidimensionnelle d'un élément de test (120) en vue de déceler au moins un analyte dans un liquide corporel, au moins un élément à lancette (114) doté d'au moins un capillaire (116) étant utilisé, le liquide corporel absorbé dans le capillaire (116) étant transféré sur l'élément de test (120), au moins une partie de l'élément de test (120) étant représentée sur une zone d'image (148) au moyen d'au moins un détecteur optique (138) à résolution locale ayant une pluralité de pixels disposés en un arrangement matriciel, le détecteur (138) possédant un côté long et un côté large, le côté long étant orienté parallèlement aux capillaires (116) du dispositif (110) et le côté large étant orienté perpendiculairement aux capillaires (116), le détecteur (138) possédant au moins 3 lignes de pixels (158) dans la direction du côté large, le détecteur (138) possédant en outre au moins une colonne de pixels (160) dans la direction du côté long, au moins une partie de la zone d'interprétation (136) de l'élément de test (120) étant représentée sur une zone d'image d'interprétation (150), la zone d'interprétation (136) étant reconnue automatiquement conformément à un procédé choisi dans le groupe composé de :
- un procédé de reconnaissance de modèle, ledit procédé de reconnaissance de modèle comprenant la reconnaissance de l'élément à lancette (114) et/ou du capillaire (116), la zone d'interprétation (136) identifiée étant une extrapolation de l'élément à lancette (114) et/ou des capillaires (116) sur l'élément de test (120) ; et
- un procédé de modification de signal, la zone d'interprétation (136) identifiée étant une zone de l'élément de test (120) à l'intérieur de laquelle, du fait du transfert du liquide corporel sur l'élément de test (120), il se produit une modification décelable optiquement.
